# EUROPÄISCHE PATENTSCHRIFT

(11) **EP 2 984 076 B1**
(45) Veröffentlichungstag und Bekanntmachung des Hinweises auf die Patenterteilung: **01.11.2017**
(21) Anmeldenummer: 14716598.9
(22) Anmeldetag: 11.04.2014
(51) Int. Cl.: C07D 307/24, C07D 307/12, C08K 5/12

(54) **2,5-TETRAHYDROFURAN-ESTER UND -ETHER ALS WEICHMACHER**
2,5-TETRAHYDROFURANE-ESTERS AND -ETHERS AS PLASTICIZERS
ESTERS ET ÉTHERS DU 2,5-TETRAHYDROFURANE EN TANT QUE PLASTIFIANTS

(30) Priorität: 12.04.2013 EP 13163577
(43) Veröffentlichungstag der Anmeldung: 17.02.2016
(73) Patentinhaber: BASF SE, 67056 Ludwigshafen am Rhein (DE)
(72) Erfinder: WAGNER, Jochen, 66957 Ruppertsweiler (DE); BREITSCHEIDEL, Boris, 67165 Waldsee (DE); BOHN, Martin Alexander, 68161 Mannheim (DE); BLANK, Benoit, 68535 Edingen-Neckarhausen (DE); KINDLER, Alois, 67269 Grünstadt (DE)
(86) Internationale Anmeldenummer: PCT/EP2014/057411
(87) Internationale Veröffentlichungsnummer: WO 2014/167108

(56) Entgegenhaltungen:
- EP-A1- 2 128 227
- WO-A1-2012/113608
- GB-A- 703 929
- JP-A- 52 013 589
- JP-A- 2004 062 011

## Beschreibung

### HINTERGRUND DER ERFINDUNG

Die vorliegende Erfindung betrifft Tetrahydrofuranderivate, eine Weichmacherzusammensetzung, die diese Tetrahydrofuranderivate enthält, Formmassen, die ein thermoplastisches Polymer und ein solches Tetrahydrofuranderivat enthalten, ein Verfahren zur Herstellung dieser Tetrahydrofuranderivate und deren Verwendung.

### STAND DER TECHNIK

Zur Erzielung gewünschter Verarbeitungs- bzw. Anwendungseigenschaften werden einer Vielzahl von Kunststoffen sogenannte Weichmacher zugesetzt, um diese weicher, flexibler und/oder dehnbarer zu machen. Im Allgemeinen dient der Einsatz von Weichmachern dazu, den thermoplastischen Bereich von Kunststoffen zu niedrigeren Temperaturen hin zu verschieben, um im Bereich niedriger Verarbeitungs- und Einsatztemperaturen die gewünschten elastischen Eigenschaften zu erhalten.

Polyvinylchlorid (PVC) gehört zu den mengenmäßig meist hergestellten Kunststoffen. Aufgrund seiner vielseitigen Anwendbarkeit findet es sich heutzutage in einer Vielzahl von Produkten des täglichen Lebens. PVC wird daher eine sehr große wirtschaftlich Bedeutung zugemessen. PVC ist ursprünglich ein bis ca. 80°C harter und spröder Kunststoff, der durch Zugabe von Thermostabilisatoren und anderen Zuschlagstoffen als Hart-PVC (PVC-U) eingesetzt wird. Erst durch die Zugabe geeigneter Weichmacher gelangt man zu Weich-PVC (PVC-P), das für viele Anwendungszwecke verwendet werden kann, für die Hart-PVC ungeeignet ist.

Weitere wichtige thermoplastische Polymere in denen üblicherweise Weichmacher Anwendung finden sind z.B. Polyvinylbutyral (PVB), Homo- und Copolymere von Styrol, Polyacrylate, Polysulfide oder thermoplastische Polyurethane (PU).

Auf dem Markt ist eine Vielzahl verschiedener Verbindungen zur Weichmachung von PVC und weiteren Kunststoffen erhältlich. Aufgrund ihrer guten Verträglichkeit mit dem PVC und ihrer vorteilhaften anwendungstechnischen Eigenschaften wurden in der Vergangenheit vielfach Phthalsäurediester mit Alkoholen unterschiedlicher chemischer Struktur als Weichmacher eingesetzt, wie z.B. Diethylhexylphthalat (DEHP), Diisononylphthalat (DINP) und Düsodecylphthalat (DIDP). Kurzkettige Phthalate, wie beispielsweise Dibutylphthalat (DBP), Düsobutylphthalat (DIBP), Benzylbutylphthalat (BBP) oder Düsoheptylphthalat (DIHP), werden auch als Gelierhilfsmittel ("fast fuser") eingesetzt, z.B. bei der Herstellung von sogenannten Plastisolen. Neben den kurzkettigen Phthalaten können auch Dibenzoesäureester wie Dipropylenglycoldibenzoate zum gleichen Zwecke eingesetzt werden. Eine weitere Klasse von Weichmachern mit guten Geliereigenschaften, sind Phenylester von Alkylsulfonsäuren, die beispielsweise als Mischungen unter dem Namen Mesamoll® TP-LXS 51067 auf dem Markt erhältlich sind.

Bei Plastisolen handelt es sich zunächst um eine Suspension von feinpulvrigen Kunststoffen in flüssigen Weichmachern. Dabei ist die Lösungsgeschwindigkeit des Polymers in dem Weichmacher bei Umgebungstemperatur sehr gering. Erst beim Erwärmen auf höhere Temperaturen löst sich das Polymer merklich im Weichmacher. Dabei quellen und fusionieren die einzelnen isolierten Kunststoffaggregate zu einem dreidimensionalen hochviskosen Gel. Dieser Vorgang wird als Gelieren bezeichnet und findet ab einer gewissen Mindesttemperatur statt, die als Gelier- oder Lösetemperatur bezeichnet wird. Der Schritt der Gelierung ist nicht reversibel.

Da Plastisole in flüssiger Form vorliegen, werden diese sehr häufig zum Beschichten von verschiedensten Materialien, wie z.B. Textilien, Glasvliesen etc. eingesetzt. Dabei wird die Beschichtung sehr häufig aus mehreren Lagen aufgebaut.
In der Praxis wird bei der Verarbeitung von Plastisolprodukten deshalb oftmals so vorgegangen, dass eine Schicht Plastisol aufgebracht wird und direkt im Anschluss der Kunststoff, insbesondere PVC, mit dem Weichmacher oberhalb der Lösetemperatur angeliert wird, also eine feste Schicht bestehend aus einer Mischung aus gelierten, teilweise gelierten und nicht-gelierten Kunststoffpartikeln entsteht. Auf diese angelierte Schicht wird dann die nächste Lage aufgebracht und nach Aufbringen der letzten Schicht der Gesamtaufbau durch Erwärmen auf höhere Temperaturen komplett zum vollständig gelierten Kunststoffprodukt verarbeitet.

Neben Plastisolen können auch trockene pulverförmige Mischungen aus Weichmacher und Kunststoffen hergestellt werden. Solche Dry-Blends, insbesondere auf Basis PVC, können dann bei erhöhten Temperaturen z.B. durch Extrusion zu einem Granulat weiterverarbeitet oder durch herkömmliche Formgebungsverfahren, wie Spritzgießen, Extrudieren oder Kalandrieren, zum vollständig gelierten Kunststoffprodukt verarbeitet werden.

Insbesondere bei der Herstellung und Verarbeitung von PVC-Plastisolen, beispielsweise zur Herstellung von PVC-Beschichtungen, ist es unter anderem wünschenswert, einen Weichmacher mit möglichst niedriger Geliertemperatur und niedriger Viskosität als Gelierhilfsmittel zur Verfügung zu haben. Darüber hinaus ist auch eine hohe Lagerstabilität des Plastisols gewünscht, d.h. das nicht-gelierte Plastisol soll bei Umgebungstemperatur keinen oder nur einen geringen Viskositätsanstieg mit der Zeit aufweisen. Diese Eigenschaften sollen möglichst durch Zugabe eines geeigneten Weichmachers mit Schnellgeliereigenschaften erzielt werden, wobei sich der Einsatz von weiteren viskositätsverringernden Additiven und/oder von Lösungsmitteln erübrigen soll.

Zum Einstellen der gewünschten Eigenschaften ist auch bekannt, Mischungen von Weichmachern einzusetzen, z. B. wenigstens einen Weichmacher, der gute thermoplastische Eigenschaften verleiht, aber schlecht geliert, in Kombination mit wenigstens einem Gelierhilfsmittel.

Es besteht der Bedarf, die eingangs erwähnten Phthalat-Weichmacher zu ersetzen, da diese toxikologisch nicht ganz unbedenklich sind. Dies gilt speziell für sensible Anwendungsbereiche wie Kinderspielzeug, Lebensmittelverpackungen oder medizinische Artikel.

Im Stand der Technik sind verschiedene alternative Weichmacher für diverse Kunststoffe und speziell für PVC bekannt.

Eine aus dem Stand der Technik bekannte Weichmacherklasse, die als Alternative zu Phthalaten eingesetzt werden kann, basiert auf Cyclohexanpolycarbonsäuren, wie sie in der WO 99/32427 beschrieben sind. Im Gegensatz zu ihren unhydrierten aromatischen Analoga sind diese Verbindungen toxikologisch unbedenklich und können auch in sensiblen Anwendungsbereichen eingesetzt werden. Die entsprechenden Niederalkylester besitzen in der Regel schnellgelierende Eigenschaften.

Die WO 00/78704 beschreibt ausgewählte Dialkylcyclohexan-1,3- und 1,4-dicarbonsäureester für die Verwendung als Weichmacher in synthetischen Materialien.

Die US 7,973,194 B1 lehrt die Verwendung von Dibenzylcyclohexan-1,4-dicarboxylat, Benzylbutylcyclohexan-1,4-dicarboxylat und Dibutylcyclohexan-1,4-dicarboxylat als schnellgelierende Weichmacher für PVC.

Einige Dietherderivate von 2,5-Di(hydroxymethyl)tetrahydrofuran sind bereits stofflich bekannt. Die WO 2009/141166 beschreibt eine Kraftstoffzusammensetzung bestehend aus ringhydrierten Alkylfurfurylethern der allgemeinen Formel: R"-TF-CH₂-O-R, worin TF ein 2,5-disubstituierter Tetrahydrofuranring, R eine Hydrocarbylgruppe mit 1 bis 20 C-Atomen, R" eine Methylgruppe, eine Hydroxymethylgruppe sowie das Produkt einer Aldolkondensation repräsentiert oder eine Alkoxymethylgruppe der allgemeinen Formel: - CH₂-O-R', worin R' eine Hydrocarbylgruppe mit 1 bis 20 C-Atomen repräsentiert. Konkret werden als Rest R und R' nur Methyl und Ethyl eingesetzt. Dieses Dokument beansprucht die stoffliche Neuheit dieser Verbindungen und beschreibt auch einen Prozess zu deren Herstellung, lehrt jedoch nur deren Einsatz als Kraftstoff oder Kraftstoffadditive und nicht als Weichmacher.

Eine weitere Weichmacherklasse sind die Ester der 2,5-Furandicarbonsäure (FDCA).
Die WO 2012/113608 beschreibt C₅-Dialkylester der 2,5-Furandicarbonsäure und deren Verwendung als Weichmacher. Diese kurzkettigen Ester eignen sich speziell auch zur Herstellung von Plastisolen.
Die WO 2012/113609 beschreibt C₇-Dialkylester der 2,5-Furandicarbonsäure und deren Verwendung als Weichmacher.

Die WO 2011/023490 beschreibt C₉-Dialkylester der 2,5-Furandicarbonsäure und deren Verwendung als Weichmacher.
Die WO 2011/023491 beschreibt C₁₀-Dialkylester der 2,5-Furandicarbonsäure und deren Verwendung als Weichmacher.
EP 2 128 227 A1 beschreibt die Herstellung von 2-(Alkoxymethyl)furanderivaten und deren Verwendung als Treibstoffzusatz.
JP 2004 062011 A beschreibt 2,5-Tetrahydrofurncarbonsäureester mit tert. Alkylgruppen, tert. Cycloalkylgruppen oder tert. Alkoxylgruppen und deren Verwendung als Additiv in Fotolacken.

JP 52 01359 A offenbart unter anderem das 2,5-Dibutoxytetrahydrofuran und dessen Verwendung in der Nachbehandlung von Polyolefinen. GB 703,929 beschreibt ein Verfahren zur Herstellung von 2,5-Dialkoxytetrahydrofuranen.
R. D. Sanderson et al. (J. Appl. Pol. Sci., 1994, Vol. 53, 1785-1793) beschreiben die Synthese von Estern der 2,5-Furandicarbonsäure und deren Verwendung als Weichmacher für Kunststoffe, insbesondere Polyvinylchlorid (PVC), Polyvinylbutyral (PVB), Polymilchsäure (PLA), Polyhydroxybuttersäure (PHB) oder Polyalkylmethacrylat (PA-MA). Konkret werden die Di(2-ethylhexyl)-, Di(2-octyl)-, Dihexyl- und Dibutylester der 2,5-Furandicarbonsäure beschrieben und deren weichmachenden Eigenschaften über dynamisch mechanische Thermoanalysen charakterisiert.
Die US 3,259,636 beschreibt ein Verfahren zur Herstellung von Estern der cis-2,5-Tetrahydrofurandicarbonsäure, bei dem man in einer Eintopfreaktion Wasserstoff, 2,5-Furandicarbonsäure und einen Alkohol in Gegenwart eines Edelmetallkatalysators umsetzt. Es ist weiterhin offenbart, dass sich die Ester von Alkoholen mit 6 oder mehr Kohlenstoffatomen als Weichmacher in Harzzusammensetzungen eignen.

Der vorliegenden Erfindung liegt die Aufgabe zugrunde, neue Verbindungen zur Verfügung zu stellen, die vorteilhaft als oder in Weichmachern für thermoplastische Polymere und Elastomere eingesetzt werden können. Sie sollen toxikologisch unbedenklich sein und sich aus gut zugängigen Edukten herstellen lassen, die vorzugsweise zumindest teilweise aus nachwachsenden Rohstoffen stammen. Sie sollen gute Geliereigenschaften besitzen und/oder eine geringe Viskosität im nicht gelierten Zustand aufweisen und sich dadurch insbesondere zur Bereitstellung von Plastisolen eignen. Die neuen Verbindungen sollten demnach in der Lage sein, die derzeitig vorherrschenden Standardweichmacher auf petrochemischer Basis zumindest gleichwertig zu ersetzen.

Diese Aufgabe wird überaschenderweise gelöst durch die Verwendung von Tetrahydrofuranderivate der allgemeinen Formel (I), 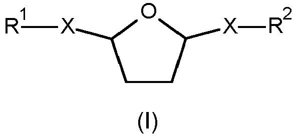 worin
- X: für ∗-(C=O)-O-, ∗-(CH₂)ₙ-O- oder ∗-(CH₂)ₙ-O-(C=O)- steht, wobei ∗ den Verknüpfungspunkt mit dem Tetrahydrofuranring darstellt und n den Wert 0, 1 oder 2 aufweist;
und
R¹ und R² unabhängig voneinander ausgewählt sind unter C₄-C₅-Alkyl und C₅-C₆-Cycloalkyl, wobei die Cycloalkylreste unsubstituiert sind oder durch wenigstens einen C₁-C₁₀-Alkylrest substituiert sein können,
oder einer Weichmacher-Zusammensetzung, enthaltend wenigstens eine Verbindung der allgemeinen Formel (I), wie oben definiert, und wenigstens einen von den Verbindungen der Formel (I) verschiedenen Weichmacher, als Weichmacher für thermoplastische Polymere und Elastomere.
Ein weiterer Gegenstand der Erfindung sind Verbindungen der allgemeinen Formel (I), 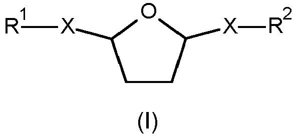 worin
X für *-(C=O)-O-, *-(CH₂)₂-O- oder *-(CH₂)-O-(C=O)- steht, wobei * den Verknüpfungspunkt mit dem Tetrahydrofuranring darstellt; und R¹ und R² unabhängig voneinander ausgewählt sind unter n-Butyl und Isobutyl.

Ein weiterer Gegenstand der Erfindung sind Verfahren zur Herstellung von Verbindungen der allgemeinen Formel (I), wobei R¹ und R² unabhängig voneinander ausgewählt sind unter n-Butyl oder Isobutyl.
Ein weiterer Gegenstand der Erfindung ist die Verwendung von Verbindungen der allgemeinen Formel (I) als oder in Weichmachern für thermoplastische Polymere, insbesondere Polyvinylchlorid (PVC).

Ein weiterer Gegenstand der Erfindung sind Formmassen, die wenigstens ein thermoplastisches Polymer, und wenigstens eine Verbindung der allgemeinen Formel (I), wie zuvor und im Folgenden definiert, enthalten.

Ein weiterer Gegenstand der Erfindung ist die Verwendung dieser Formmassen zur Herstellung von Formkörpern und Folien.

### BESCHREIBUNG DER ERFINDUNG

Die erfindungsgemäßen Verbindungen (I) weisen die folgenden Vorteile auf:
- Die erfindungsgemäßen Verbindungen (I) eignen sich aufgrund ihrer physikalischen Eigenschaften sehr gut für die Anwendungen als Weichmacher oder als Komponente einer Weichmacher-Zusammensetzung für thermoplastische Polymere, insbesondere für PVC.
- Die erfindungsgemäßen Verbindungen (I) eignen sich aufgrund ihrer niedrigen Lösetemperaturen nach DIN 53408 sehr gut als Gelierhilfsmittel. Sie eignen sich somit zum Verringern der zum Gelieren eines thermoplastischen Polymers erforderlichen Temperatur und/oder zur Erhöhung der Geliergeschwindigkeit.
- Die erfindungsgemäßen Verbindungen der allgemeinen Formel (I) zeichnen sich durch eine sehr gute Verträglichkeit mit einer Vielzahl verschiedener Weichmacher aus. Sie eignen sich speziell in Kombination mit konventionellen Weichmachern zur Verbesserung des Gelierverhaltens.
- Die erfindungsgemäßen Verbindungen (I) eignen sich in vorteilhafter Weise zur Herstellung von Plastisolen.
- Die erfindungsgemäßen Verbindungen (I) eignen sich für die Verwendung zur Herstellung von Formkörpern und Folien für sensible Anwendungsbereiche, wie Medizinprodukte, Lebensmittelverpackungen, Produkte für den Innenraumbereich, beispielsweise von Wohnungen und Fahrzeugen, Spielzeuge, Kinderpflegeartikel, etc.
- Zur Herstellung der erfindungsgemäßen Verbindungen (I) können leicht zugängliche Edukte verwendet werden. Ein besonderer ökonomischer und ökologischer Vorteil der vorliegenden Erfindung liegt in der Möglichkeit, bei der Herstellung der erfindungsgemäßen Verbindungen (I) sowohl in großen Mengen zur Verfügung stehende petrochemische Rohstoffe, wie auch nachwachsende Rohstoffe nutzen zu können. So sind beispielsweise die Ausgangsstoffe der Furankerne aus natürlich vorkommenden Kohlenhydraten, wie Cellulose und Stärke, erhältlich, wohingegen die zur Einführung der Seitenketten einsetzbaren Alkohole aus großtechnischen Verfahren zur Verfügung stehen. So kann einerseits der Bedarf an "nachhaltigen" Produkten gedeckt werden, anderseits ist aber auch eine wirtschaftliche Herstellung möglich.
- Die Verfahren zur Herstellung der erfindungsgemäßen Verbindungen (I) sind einfach und effizient, wodurch diese problemlos in großtechnischem Maßstab bereitgestellt werden können.

Wie zuvor erwähnt wurde überaschenderweise festgestellt, dass die Verbindungen der allgemeinen Formel (I), insbesondere die C₄-C₅-Dialkylester der Tetrahydrofurandicarbonsäure, sehr niedrige Lösetemperaturen sowie exzellente Geliereigenschaften bei der Herstellung von Plastisolen, insbesondere von PVC-Plastisolen, aufweisen. So liegen deren Lösetemperaturen deutlich unter den Lösetemperaturen der entsprechenden Dialkylestern der 2,5-Furandicarbonsäure oder Phthalsäure und besitzen mindestens ebenbürtige schnellgelierende Eigenschaften. Dies war nicht zu erwarten, da beispielsweise kernhydrierte Phthalate, wie Diisononylcyclohexan-1,2-dicarboxylat, in der Regel höhere Lösetemperaturen aufweisen als ihre unhydrierten Formen. So weist z.B. der 1,2-Cyclohexandicarbonsäure-diisononylester mit 151°C eine höhere Lösetemperatur nach DIN 53408 auf als Diisononylphthalat mit 132°C.

Im Rahmen der vorliegenden Erfindung wird unter einem Gelierhilfsmittel ein Weichmacher verstanden, der eine Lösetemperatur nach DIN 53408 von unter 120°C aufweist. Solche Gelierhilfsmittel werden insbesondere zur Herstellung von Plastisolen verwendet.

Die erfindungsgemäßen Verbindungen der allgemeinen Formel (I.1) können sowohl reine cis-Isomere oder als reine trans-Isomere oder als cis/trans-Isomerengemische vorliegen. Sowohl die reinen Isomere als auch die Isomerengemische beliebiger Zusammensetzung eignen sich in gleichem Maße als Weichmacher.

Im Rahmen der vorliegenden Erfindung umfasst der Ausdruck "C₁-C₁₀-Alkyl" geradkettige oder verzweigte C₁-C₁₀-Alkylgruppen. Vorzugsweise handelt es sich dabei um geradkettige oder verzweigte C₁-C₈-Alkylgruppen. Dazu zählen Methyl, Ethyl, Propyl, Isopropyl, n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl und dergleichen. Besonders bevorzugt handelt es sich dabei um geradkettige oder verzweigte C₁-C₅-Alkylgruppen.

Der Ausdruck "C₄-C₅-Alkyl" umfasst geradkettige und verzweigte C₄-C₅-Alkylgruppen. Vorzugsweise ist C₄-C₅-Alkyl ausgewählt unter n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl und 1-Ethylpropyl. Besonders bevorzugt steht C₄-C₅-Alkyl für n-Butyl, Isobutyl oder n-Pentyl.

Der Ausdruck "C₅-C₆-Cycloalkyl" umfasst im Sinne der vorliegenden Erfindung cyclische Kohlenwasserstoffe mit 5 bis 6, insbesondere mit 6 Kohlenstoffatomen. Dazu zählen Cyclopentyl oder Cyclohexyl.

Substituierte C₅-C₆-Cycloalkylgruppen können, in Abhängigkeit von ihrer Ringgröße, einen oder mehrere (z. B. 1, 2, 3, 4 oder 5) C₁-C₁₀-Alkylsubstituenten aufweisen. Beispiele für C₅-C₆-Cycloalkylgruppen sind 2- und 3-Methylcyclopentyl, 2- und 3-Ethylcyclopentyl, 2-, 3- und 4-Methylcyclohexyl, 2-, 3- und 4-Ethylcyclohexyl, 2-, 3- und 4-Propylcyclohexyl, 2-, 3- und 4-lsopropylcyclohexyl, 2-, 3- und 4-Butylcyclohexyl, 2-, 3- und 4-sec.-Butylcyclohexyl und 2-, 3- und 4-tert.-Butylcyclohexyl.

Vorzugsweise weisen die Gruppen X in den Verbindungen der allgemeinen Formel (I) dieselbe Bedeutung auf.

In einer ersten bevorzugten Ausführungsform steht in den Verbindungen der allgemeinen Formel (I) die Gruppen X beide für ∗-(C=O)-O-.

In einer weiteren bevorzugten Ausführungsform stehen in den Verbindungen der allgemeinen Formel (I) die Gruppen X beide für ∗-(CH₂)-O-(C=O)-.

In einer weiteren bevorzugten Ausführungsform stehen in den Verbindungen der allgemeinen Formel (I) die Gruppen X beide für ∗-(CH₂)ₙ-O-, wobei n für 0, 1 oder 2 steht. Besonders bevorzugt steht n für 2.

Bevorzugt stehen in den Verbindungen der allgemeinen Formel (I) die Reste R¹ und R² unabhängig voneinander für einen unverzweigten oder verzweigten C₄-Alkylrest.

Besonders bevorzugt stehen in den Verbindungen der allgemeinen Formel (I) die Reste R¹ und R² unabhängig voneinander für n-Butyl oder Isobutyl.

In einer bevorzugten Ausführung haben in den Verbindungen der allgemeinen Formel (I) die Reste R¹ und R² dieselbe Bedeutung.

Bevorzugte Verbindungen der allgemeinen Formel (I) sind ausgewählt unter Di-(n-butyl)-2,5-tetrahydrofurandicarboxylat, Di-n-butylether von 2,5-Di(hydroxymethyl)tetrahydrofuran, 2,5-Di(hydroxymethyl)tetrahydrofuran-di-n-butanoat, Di-(isobutyl)-2,5-tetrahydrofurandicarboxylat, Di-isobutylether von 2,5-Di(hydroxymethyl)tetrahydrofuran, 2,5-Di(hydroxymethyl)tetrahydrofuran-di-isobutanoat
sowie Mischungen aus 2 oder mehr als 2 der zuvor genannten Verbindungen.

Eine besonders bevorzugte Verbindung der allgemeinen Formel (I) ist Di-(n-butyl)-2,5-tetrahydrofurandicarboxylat.

### Herstellung der Verbindungen der allgemeinen Formel (I)

### Herstellung der Diester der 2,5-Tetrahydrofurandicarbonsäure

Ein weiterer Gegenstand der Erfindung ist ein Verfahren zur Herstellung von Verbindungen der allgemeinen Formel (I.1), 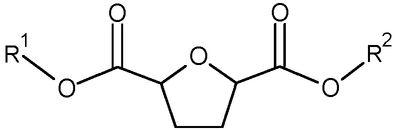 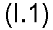 worin
R¹ und R² unabhängig voneinander ausgewählt sind unter n-Butyl oder Isobutyl, bei dem man
a) gegebenenfalls 2,5-Furandicarbonsäure oder ein Anhydrid oder Säurehalogenid davon mit einem C₁-C₃-Alkanol in Gegenwart eines Katalysators unter Erhalt eines Di-(C₁-C₃-Alkyl)-2,5-furandicarboxylats umsetzt,
b1) 2,5-Furandicarbonsäure oder ein Anhydrid oder Säurehalogenid davon oder das in Schritt a) erhaltene Di-(C₁-C₃-Alkyl)-2,5-furandicarboxylat mit n-Butanol und/oder Isobutanol in Gegenwart wenigstens eines Katalysators unter Erhalt einer Verbindung der Formel (I.1a) umsetzt, 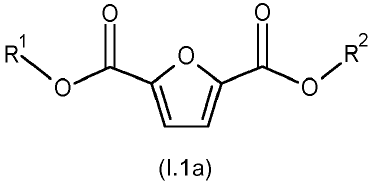
c1) die in Schritt b1) erhaltene Verbindung (I.1a) mit Wasserstoff in Gegenwart wenigstens eines Hydrierkatalysators unter Erhalt der Verbindung der allgemeinen Formel (I.1) hydriert,
   oder
b2) 2,5-Furandicarbonsäure oder das in Schritt a) erhaltene 2,5-Di-(C₁-C₃-Alkyl)furandicarboxylat mit Wasserstoff in Gegenwart wenigstens eines Hydrierkatalysators unter Erhalt einer Verbindung der allgemeinen Formel (I.1b) hydriert, 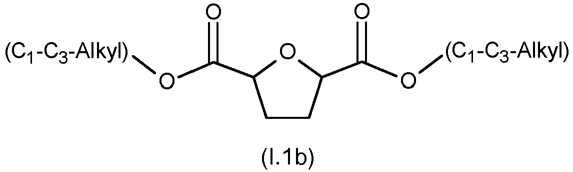
c2) die in Schritt b2) erhaltene Verbindung (I.1b) mit n-Butanol und/oder Isobutanol in Gegenwart eines Katalysators unter Erhalt einer Verbindung der Formel (I.1) umsetzt.

Das erfindungsgemäße Verfahren ermöglicht die Herstellung der 2,5-Tetrahydrofurandicarbonsäuredibutylester der allgemeinen Formel (I.1) auf zwei verschiedenen Wegen (nachfolgend als Variante 1 und Variante 2 bezeichnet).

Für den Einsatz in Schritt a) geeignete C₁-C₃-Alkanole sind z.B. Methanol, Ethanol, n-Propanol oder Gemische davon.

In Variante 1 des erfindungsgemäßen Verfahrens wird die 2,5-Furandicarbonsäure oder das in Schritt a) erhaltene Di-(C₁-C₃-Alkyl)-2,5-furandicarboxylat einer Veresterung bzw. Umesterung mit n-Butanol und/oder Isobutanol zu den Verbindungen der Formel (1.a) unterzogen, welche anschließend zu 2,5-Tetrahydrofurandicarbonsäuredibutylester allgemeinen Formel (I.1) hydriert werden (Schritt c1)).

In Variante 2 wird die 2,5-Furandicarbonsäure oder das in Schritt a) erhaltene 2,5-Di-(C₁-C₃-Alkyl)furandicarboxylat zunächst unter Erhalt von 2,5-Tetrahydrofurandicarbonsäure bzw. einer Verbindung der allgemeinen Formel (I.1b) hydriert (Schritt b2)) und das Produkt der Hydrierung anschließend mit n-Butanol und/oder Isobutanol H zu 2,5-Tetrahydrofurandicarbonsäuredibutylester der allgemeinen Formel (1.1) umgesetzt (Schritt c2)).

### Veresterung

Die Überführung der 2,5-Furandicarbonsäure (FDCS) bzw. der 2,5-Tetrahydrofurandicarbonsäure in die entsprechenden Esterverbindungen der allgemeinen Formeln (I.1), (I.1a) und (I.1b) kann nach üblichen dem Fachmann bekannten Verfahren erfolgen. Dazu zählt die Umsetzung wenigstens einer Alkoholkomponente, ausgewählt aus C₁-C₃-Alkanolen oder n-Butanol bzw. Isobutanol, mit FDCS oder einem geeigneten Derivat davon. Geeignete Derivate sind z. B. die Säurehalogenide und Säureanhydride. Ein bevorzugtes Säurehalogenid ist das Säurechlorid. Als Veresterungskatalysatoren können die dafür üblichen Katalysatoren eingesetzt werden, z. B. Mineralsäuren, wie Schwefelsäure und Phosphorsäure; organische Sulfonsäuren, wie Methansulfonsäure und p-Toluolsulfonsäure; amphotere Katalysatoren, insbesondere Titan-, Zinn (IV)- oder Zirkoniumverbindungen, wie Tetraalkoxytitane, z. B. Tetrabutoxytitan, und Zinn (IV)-oxid. Das bei der Reaktion entstehende Wasser kann durch übliche Maßnahmen, z. B. destillativ, entfernt werden. Die WO 02/038531 beschreibt ein Verfahren zur Herstellung von Estern, bei dem man a) in einer Reaktionszone ein im Wesentlichen aus der Säurekomponente oder einem Anhydrid davon und der Alkoholkomponente bestehendes Gemisch in Gegenwart eines Veresterungskatalysators zum Sieden erhitzt, b) die Alkohol und Wasser enthaltenden Dämpfe rektifikativ in eine alkoholreiche Fraktion und eine wasserreiche Fraktion auftrennt, c) die alkoholreiche Fraktion in die Reaktionszone zurück führt und die wasserreiche Fraktion aus dem Verfahren ausleitet. Als Veresterungskatalysatoren werden die zuvor genannten Katalysatoren eingesetzt. Der Veresterungskatalysator wird in einer wirksamen Menge eingesetzt, die üblicherweise im Bereich von 0,05 bis 10 Gew.-%, vorzugsweise 0,1 bis 5 Gew.-%, bezogen auf die Summe von Säurekomponente (oder Anhydrid) und Alkoholkomponente, liegt. Weitere detaillierte Darstellungen zur Durchführung von Veresterungsverfahren finden sich beispielsweise in der US 6,310,235, US 5,324,853, DE-A 2612355 (Derwent Abstract Nr. DW 77-72638 Y) oder DE-A 1945359 (Derwent Abstract Nr. DW 73-27151 U). Auf die genannten Dokumente wird in vollem Umfang Bezug genommen.

In einer bevorzugten Ausführung erfolgt die Veresterung von FDCS oder der 2,5-Tetrahydrofurandicarbonsäure in Anwesenheit der oben beschriebenen Alkoholkomponenten, mittels einer organischen Säure oder Mineralsäure, insbesondere konzentrierter Schwefelsäure. Dabei wird die Alkoholkomponente vorteilhafterweise wenigstens in der doppelten stöchiometrischen Menge bezogen auf die FDCS oder die 2,5-Tetrahydrofurandicarbonsäure oder ein Derivat eingesetzt.

Die Veresterung kann in der Regel bei Umgebungsdruck oder vermindertem oder erhöhtem Druck erfolgen. Bevorzugt wird die Veresterung bei Umgebungsdruck oder vermindertem Druck durchgeführt.

Die Veresterung kann in Abwesenheit eines zugesetzten Lösungsmittels oder in Gegenwart eines organischen Lösungsmittels durchgeführt werden.

Falls die Veresterung in Gegenwart eines Lösungsmittels durchgeführt wird, handelt es sich dabei vorzugsweise um ein unter den Reaktionsbedingungen inertes organisches Lösungsmittel. Dazu gehören beispielsweise aliphatische Kohlenwasserstoffe, halogenierte aliphatische Kohlenwasserstoffe, aromatische und substituierte aromatische Kohlenwasserstoffe oder Ether. Bevorzugt ist das Lösungsmittel ausgewählt unter Pentan, Hexan, Heptan, Ligroin, Petrolether, Cyclohexan, Dichlormethan, Trichlormethan, Tetrachlormethan, Benzol, Toluol, Xylol, Chlorbenzol, Dichlorbenzolen, Dibutylether, THF, Dioxan und Mischungen davon.

Die Veresterung wird üblicherweise in einem Temperaturbereich von 50 bis 250°C durchgeführt.

Ist der Veresterungskatalysator ausgewählt unter organischen Säuren oder Mineralsäuren, wird die Veresterung üblicherweise in einem Temperaturbereich von 50 bis 160°C durchgeführt.

Ist der Veresterungskatalysator ausgewählt unter amphoteren Katalysatoren, wird die Veresterung üblicherweise in einem Temperaturbereich von 100 bis 250°C durchgeführt.

Die Veresterung kann in Abwesenheit oder in Gegenwart eines Inertgases erfolgen. Unter einem Inertgas wir in der Regel ein Gas verstanden, welches unter den gegebenen Reaktionsbedingungen keine Reaktionen mit den an der Reaktion beteiligten Edukten, Reagenzien, Lösungsmitteln oder den entstehenden Produkten eingeht. Bevorzugt erfolgt die Veresterung ohne die Zugabe eines Inertgases.

### Umesterung:

Die in den Schritten b1) und c2) beschriebene Umesterung der Di-(C₁-C₃-Alkyl)-2,5-furandicarboxylate bzw. der Di-(C₁-C₃-Alkyl)-2,5-tetrahydrofurandicarboxylate zu den entsprechenden Esterverbindungen I.1a bzw. I.1 kann nach üblichen dem Fachmann bekannten Verfahren erfolgen. Dazu zählt die Umsetzung der Di-(C₁-C₃)-Alkylester mit n-Butanol und/oder Isobutanol in Anwesenheit eines geeigneten Umesterungskatalysators.

Als Umesterungskatalysatoren kommen die üblichen gewöhnlich für Umesterungsreaktionen verwendeten Katalysatoren in Betracht, die meist auch bei Veresterungsreaktionen eingesetzt werden. Hierzu zählen z. B. Mineralsäuren, wie Schwefelsäure und Phosphorsäure; organische Sulfonsäuren, wie Methansulfonsäure und p-Toluolsulfonsäure; oder spezielle Metallkatalysatoren aus der Gruppe der Zinn (IV)-Katalysatoren, beispielsweise Dialkylzinndicarboxylate wie Dibutylzinndiacetat, Trialkylzinnalkoxide, Monoalkylzinnverbindungen wie Monobutylzinndioxid, Zinnsalze wie Zinnacetat oder Zinnoxide; aus der Gruppe der Titankatalysatoren, monomere und polymere Titanate und Titanchelate wie Tetraethylorthotitanat, Tetrapropylorthotitanat, Tetrabutylorthotitanat, Triethanolamintitanat; aus der Gruppe der Zirkonkatalysatoren, Zirkonate und Zirkonchelate wie Tetrapropylzirkonat, Tetrabutylzirkonat, Triethanolaminzirkonat; sowie Lithiumkatalysatoren wie Lithiumsalze, Lithiumalkoxide; oder Aluminium(III)-, Chrom(III)-, Eisen(III)-, Kobalt(II)-, Nickel(II) und Zink(II)-acetylacetonat.

Die Menge an eingesetztem Umesterungskatalysator liegt bei 0,001 bis 10 Gew.-%, bevorzugt bei 0,05 bis 5 Gew.-%. Das Reaktionsgemisch wird bevorzugt bis zum Siedepunkt des Reaktionsgemisches erhitzt, sodass die Reaktionstemperatur in Abhängigkeit von den Reaktanten zwischen 20°C und 200°C liegt.

Die Umesterung kann bei Umgebungsdruck oder vermindertem oder erhöhtem Druck erfolgen. Bevorzugt wird die Umesterung bei einem Druck von 0,001 bis 200 bar, besonders bevorzugt 0,01 bis 5 bar durchgeführt. Der bei der Umesterung abgespaltene, niedriger siedende Alkohol wird zwecks Verschiebung des Gleichgewichts der Umesterungsreaktion bevorzugt kontinuierlich abdestilliert. Die hierzu benötigte Destillationskolonne steht in der Regel in direkter Verbindung mit dem Umesterungsreaktor, vorzugsweise ist sie direkt an diesem installiert. Im Falle der Verwendung mehrerer in Serie geschalteter Umesterungsreaktoren, kann jeder dieser Reaktoren mit einer Destillationskolonne ausgerüstet sein oder es kann, vorzugsweise aus den letzten Kesseln der Umesterungsreaktorkaskade das abgedampfte Alkoholgemisch über eine oder mehrere Sammelleitungen einer Destillationskolonne zugeführt werden. Der bei dieser Destillation zurückgewonnene höhersiedende Alkohol wird vorzugsweise wieder in die Umesterung zurückgeführt.

Im Falle der Verwendung eines amphoteren Katalysators gelingt dessen Abtrennung im Allgemeinen durch Hydrolyse und anschließende Abtrennung des gebildeten Metalloxids, z. B. durch Filtration. Bevorzugt wird nach erfolgter Reaktion der Katalysator mittels Waschen mit Wasser hydrolysiert und das ausgefallene Metalloxid abfiltriert. Gewünschtenfalls kann das Filtrat einer weiteren Aufarbeitung zur Isolierung und/oder Reinigung des Produkts unterzogen werden. Bevorzugt wird das Produkt destillativ abgetrennt.

In einer bevorzugten Ausführungsform der Schritte 1b) und 2c) erfolgt die Umesterung der Di-(C₁-C₃-Alkyl)-2,5-Furandicarboxylate bzw. Di-(C₁-C₃-Alkyl)-2,5-Tetrahydrofurandicarboxylate in Anwesenheit der Alkoholkomponente und in Gegenwart wenigstens eines Titan (IV)-Alkoholats. Bevorzugte Titan (IV)-Alkoholate sind Tetrapropoxytitan, Tetrabutoxytitan oder Gemische davon. Bevorzugt wird die Alkoholkomponente wenigstens in der doppelten stöchiometrischen Menge bezogen auf die eingesetzten Di-(C₁-C₃-Alkyl)-Ester eingesetzt.

Die Umesterung kann in Abwesenheit oder in Gegenwart eines zugesetzten organischen Lösungsmittels durchgeführt werden. Bevorzugt wird die Umesterung in Gegenwart eines inerten organischen Lösungmittels durchgeführt. Geeignete organische Lösungmittel sind die zuvor für die Veresterung genannten. Dazu zählen speziell Toluol und THF.

Die Temperatur bei der Umesterung liegt vorzugsweise in einem Bereich von 50 bis 200°C.

Die Umesterung kann in Abwesenheit oder in Gegenwart eines Inertgases erfolgen. Unter einem Inertgas wir in der Regel ein Gas verstanden, welches unter den gegebenen Reaktionsbedingungen keine Reaktionen mit den an der Reaktion beteiligten Edukten, Reagenzien, Lösungsmitteln oder den entstehenden Produkten eingeht. Bevorzugt wird die Umesterung ohne Hinzufügen eines Inertgases durchgeführt.

### Hydrierung

Für die in den erfindungsgemäßen Schritten c1) und b2) durchgeführten Hydrierung der Doppelbindungen des Furankerns stehen dem Fachmann eine Reihe von Verfahren und Katalysatoren zur Verfügung, die beispielsweise auch bei der Hydrierung von Estern aromatischer Polycarbonsäuren wie Phthalaten, Isophthalaten oder Terephthalaten zum Einsatz kommen. Geeignet ist beispielsweise das in der WO 99/032427 beschriebene Kernhydrierungsverfahren. Dieses umfasst eine Hydrierung bei 50 bis 250°C und einem Druck von 20 bis 300 bar mittels Katalysatoren, die wenigstens ein Metall der VIII. Nebengruppe des Periodensystems der Elemente, beispielsweise Platin, Rhodium, Palladium, Kobalt, Nickel oder Ruthenium, bevorzugt Ruthenium, entweder alleine oder zusammen mit wenigstens einem Metall aus der I. oder VII. Nebengruppe des Periodensystems, wie Kupfer oder Rhenium abgeschieden auf einem mesoporösen Aluminiumoxid-Trägermaterial mit bimodaler Porenverteilung enthalten. Geeignet ist weiterhin das in der WO 02/100536 beschriebene Kernhydrierungsverfahren. Dieses umfasst eine Hydrierung unter Verwendung eines Rutheniumkatalysators auf amorphem Siliziumdioxid als Träger. Weitere geeignete Verfahren sind in folgenden Dokumenten beschrieben: EP-A 1266882 - Verwendung eines Nickel/Magnesiumoxid auf Kieselgur-Katalysators, WO 03/029181 - Verwendung eines Nickel/Zink auf Siliziumdioxid-Katalysators, WO 03/029168 - Verwendung eines Palladium/ZnO auf Tonerde-Katalysators und eines Ruthenium/ZnO auf α-Al₂O₃-Katalysators oder WO 04/09526 - Verwendung eines Ruthenium auf Titandioxid-Katalysators. Weitere geeignete Katalysatoren sind ebenfalls Raney-Katalysatoren, bevorzugt Raney-Nickel. Neben den bereits erwähnten Trägermaterialien eignen sich beispielsweise auch Zirkondioxid (ZrO₂), sulfatiertes Zirkondioxid, Wolframcarbid (WC), Titandioxid (TiO₂), sulfatierter Kohlenstoff, Aktivkohle, Aluminiumphosphat, Alumosilicate oder phosphatiertes Aluminiumoxid sowie Kombinationen davon.

Die Hydrierung kann in Analogie zu den bekannten Hydrierverfahren zur Hydrierung von organischen Verbindungen, die hydrierbare Gruppen aufweisen, erfolgen. Hierzu wird die organische Verbindung als flüssige Phase oder Gasphase, bevorzugt als flüssige Phase, mit dem Katalysator in Gegenwart von Wasserstoff in Kontakt gebracht. Die flüssige Phase kann z.B. über ein Katalysator-Fließbett (Fließbett-Fahrweise) oder ein Katalysator-Festbett (Festbettfahrweise) geleitet werden.

In dem erfindungsgemäßen Verfahren erfolgt die Hydrierung bevorzugt in einem Festbettreaktor.

Die Hydrierung kann sowohl kontinuierlich als auch diskontinuierlich ausgestaltet werden, wobei die kontinuierliche Verfahrensausführung bevorzugt ist. Zur diskontinuierlichen Hydrierung kann eine dafür übliche Reaktionsvorrichtung, z.B. ein Rührreaktor eingesetzt werden. Vorzugsweise führt man die erfindungsgemäße Hydrierung kontinuierlich Festbettreaktoren in der Sumpffahrweise oder Rieselfahrweise durch. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung am Katalysatorfließbett und am Katalysatorfestbett sind im Stand der Technik bekannt, z.B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM.

Die Hydrierung erfolgt in der Regel unter erhöhtem Wasserstoffdruck. Bevorzugt ist ein Wasserstoffdruck im Bereich von 2 bis 500 bar, besonders bevorzugt von 10 bis 300 bar.

Die Hydrierung erfolgt bevorzugt in Gegenwart eines unter den Hydrierbedingungen inerten organischen Lösungsmittels. Geeignete Lösungsmittel sind die zuvor bei der Veresterung definierten. Speziell wird ein Ether, wie THF, ein Dialkylenglycol oder ein Mono- oder Diether davon, wie Glyme, eingesetzt.

Die Hydrierung wird bei einer Temperatur in einem Bereich von 20 bis 350°C, besonders bevorzugt von 50 bis 300°C, durchgeführt.

Die zur Hydrierung eingesetzte Wasserstoffmenge entspricht in der Regel der 1- bis 15-fachen Menge der zur vollständigen Hydrierung des Furankerns theoretisch erforderlichen, stöchiometrischen Wasserstoffmenge.

In einer bevorzugten Ausführung der Schritte c1) und b2) wird die Hydrierung des Furankerns mit Platin, Rhodium, Palladium, Kobalt, Nickel oder Ruthenium, insbesondere Platin und Palladium, abgeschieden auf Aluminiumoxid, Zirkondioxid, sulfatiertem Zirkondioxid, Zinkoxid oder Siliziumdioxid, insbesondere Zirkondioxid, in Gegenwart eines inerten Lösungsmittels unter 150 bis 300 bar Wasserstoffdruck bei einer Temperatur von 150 bis 250°C durchgeführt.

Bei den beschriebenen Hydrierverfahren können je nach gewählter Hydrierbedingung, wie Katalysatorzusammensetzung oder der Hydriertemperatur, bevorzugt das cis- oder das trans-Isomer der 2,5-Tetrahydrofurandicarbonsäureester gebildet werden. So lassen sich im Wesentlichen isomerenreine cis- bzw. trans-2,5-Tetrahydrofurandicarbonsäureester generieren, aber auch Mischungen mit unterschiedlich hohen cis- bzw. trans-Isomerenanteilen. Unter im Wesentlichen isomerenrein wird dabei ein Gehalt von wenigstens 95 Gew.-% eines bestimmten Isomers, bezogen auf das Gesamtgewicht des 2,5-Tetrahydrofurandicarbonsäureesters, verstanden.

Die erfindungsgemäßen Verbindungen der allgemeinen Formel (I.1) können demnach sowohl als reine cis-Isomere oder als reine trans-Isomere oder als cis/trans-Isomerengemische vorliegen. Sowohl die reinen Isomere als auch die Isomerengemische beliebiger Zusammensetzung eignen sich in gleichem Maße als Weichmacher.

In einer besonders bevorzugten Ausführungsform der Schritte c1) und b2) wird FDCS bzw. die Ester der 2,5-Furandicarbonsäure aus den Schritten a) und b1) in einem inerten Lösungsmittel gelöst und in Gegenwart eines heterogenen Pd/Pt-Katalysator bei 50 bis 300 bar Wasserstoffdruck und bei 100 bis 250°C vollständig hydriert. Dabei erfolgt die Hydrierung bevorzugt kontinuierlich in der Festbettfahrweise, wobei der Wasserstoff im Gegenstrom über den Katalysator geleitet wird. Nach dieser Ausführung wird vorzugsweise THF als Lösungsmittel eingesetzt. Nach dieser Ausführung wird weiterhin vorzugsweise ein Pd/Pt-Katalysator auf ZrO₂ eingesetzt. Die bevorzugte Reaktionstemperatur dieser Ausführungsform liegt im Bereich von 100 bis 200°C. Nach dieser Ausführungsform werden im Allgemeinen die gewünschten Tetrahydrofuranderivate mit einem cis-Isomerenanteil von mindestens 90 Gew.-%, bezogen auf die Gesamtmenge der gebildeten cis/trans-Isomere, erhalten.

Eine besonders bevorzugte Ausführungsform des erfindungsgemäßen Verfahrens umfaßt:
a) Umsetzung von 2,5-Furandicarbonsäure mit Methanol in Gegenwart von konzentrierter Schwefelsäure unter Erhalt des 2,5-Furandicarbonsäuredimethylesters,
2b) Hydrierung des in Schritt a) erhaltenen 2,5-Furandicarbonsäuredimethylesters mit Wasserstoff in Gegenwart eines Pd/Pt-Katalysators auf ZrO₂ unter Erhalt des 2,5-Tetrahydrofurandicarbonsäuredimethylesters,
2c) Umsetzung des in Schritt 2b) erhaltenen 2,5-Tetrahydrofurandicarbonsäuredimethylesters mit n-Butanol und/oder Isobuntaol in Gegenwart wenigstens eines Titan (IV)-Alkoholats zu den Verbindungen der allgemeinen Formel (I.1).

Herstellung der C₄- Diether- bzw. C₄- Diesterderivate der Formel (I.2) bzw. (I.3)

Ein weiterer Gegenstand der Erfindung ist ein Verfahren zur Herstellung von Verbindungen der allgemeinen Formel (I.2) oder (I.3), 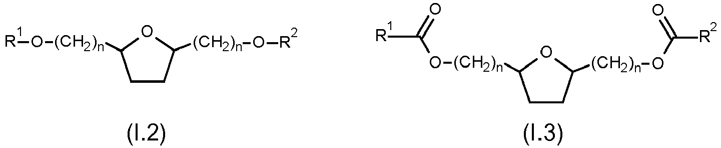 worin
R¹ und R² unabhängig voneinander ausgewählt sind unter n-Butyl oder Isobutyl, bei dem man
für 2,5-Di-(hydroxymethyl)tetrahydrofuran (n = 1) oder für 2,5-Di-(hydroxyethyl)-tetrahydrofuran (n = 2) mit wenigstens einem Alkylierungsreagenz R¹-Z und, falls R¹ und R² unterschiedliche Bedeutung haben, zusätzlich mit wenigstens einem Alkylierungsreagenz R²-Z, wobei Z für eine Abgangsgruppe steht, in Gegenwart einer Base zu Verbindungen der Formel (I.2) umsetzt,
oder
für 2,5-Di-(hydroxymethyl)tetrahydrofuran (n = 1) oder für 2,5-Di-(hydroxyethyl)-tetrahydrofuran (n = 2) mit wenigstens einem Säurehalogenid R¹-(C=O)X und, falls R¹ und R² unterschiedliche Bedeutung haben, zusätzlich mit wenigstens einem Säurehalogenid R²-(C=O)X, wobei X für Br oder Cl steht, in Gegenwart wenigstens eines tertiären Amins in Verbindungen der Formel (I.3) umgesetzt.

In der Regel wird die Alkylierung in Gegenwart eines unter den Reaktionsbedingungen inerten organischen Lösungsmittels durchgeführt. Geeignete Lösungsmittel sind die zuvor bei der Veresterung genannten. Bevorzugtes Lösungsmittel sind aromatische Kohlenwasserstoffe, wie Toluol.

Die Abgangsgruppe Z steht bevorzugt für einen Rest, der ausgewählt ist unter Br, Cl, der Tosyl-, Mesyl- oder Triflyl-Gruppe.

Besonders bevorzugt steht die Abgangsgruppe Z für Br.

Die Alkylierungsreagenzien R¹-Z bzw. R²-Z sind im Handel erhältlich oder können über geeignete dem Fachmann geläufige Reaktionen oder Verfahrensweisen aus den entsprechenden Alkoholen hergestellt werden. Beispielsweise lassen sich die für dieses Verfahren bevorzugt verwendeten Alkylbromide R¹-Br bzw. R²-Br in bekannter Weise großtechnisch unter Verwendung von Bromwasserstoff (HBr) aus den entsprechenden Alkoholen R¹-OH bzw. R²-OH herstellen.

Als geeignete Basen kommen bei dem erfindungsgemäßen Verfahren mineralische und/oder starke organische Basen in Betracht. Dazu gehören z. B. anorganische Basen oder Basenbildner, wie Hydroxide, Hydride, Amide, Oxide und Carbonate der Alkali- und Erdalkalimetalle. Dazu gehören LiOH, NaOH, KOH, Mg(OH)₂, Ca(OH)₂, LiH, NaH, Natriumamid (NaNH₂), Lithiumdiisopropylamid (LDA), Na₂O, K₂CO₃, Na₂CO₃ und Cs₂CO₃; sowie metallorganische Verbindungen wie n-BuLi oder tert.-BuLi. Bevorzugt sind NaOH, KOH, K₂CO₃ und Na₂CO₃.

Die Base wird dabei bevorzugt in einem wenigsten zweifachen stöchiometrischen Überschuss bezogen auf das 2,5-Di-(hydroxymethyl)tetrahydrofuran bzw. 2,5-Di-(hydroxyethyl)tetrahydrofuran eingesetzt. Besonders bevorzugt wird ein wenigstens vierfacher stöchiometrischer Überschuss an Base verwendet.

Die Alkylierung kann in Abwesenheit oder in Gegenwart eines organischen Lösungsmittels durchgeführt werden. In der Regel wird die Reaktion in Gegenwart eines inerten organischen Lösungsmittels, wie Pentan, Hexan, Heptan, Ligroin, Petrolether, Cyclohexan, Dichlormethan, Trichlormethan, Tetrachlormethan, Benzol, Toluol, Xylol, Chlorbenzol, Dichlorbenzole, Dibutylether, THF, Dioxan und Mischungen davon, durchgeführt.

Die Alkylierung kann in der Regel bei Umgebungsdruck, vermindertem Druck oder erhöhtem Druck erfolgen. Bevorzugt wird die Alkylierung bei Umgebungsdruck durchgeführt.

Bevorzugt wird die Alkylierung in einem Temperaturbereich von 30 bis 200°C, bevorzugt 50 bis 150°C, durchgeführt.

Die Alkylierung kann in Abwesenheit oder in Gegenwart eines Inertgases erfolgen. Bevorzugt wird bei der Alkylierung kein Inertgas eingesetzt.

In einer speziellen Ausführungsform der Alkylierung wird 2,5-Di-(hydroxymethyl)-tetrahydrofuran bzw. 2,5-Di-(hydroxyethyl)tetrahydrofuran in Gegenwart eines wenigstens vierfachen Überschusses an Base in einem inerten organischen Lösungsmittel und mit wenigstens einem Alkylbromid R¹-Br bzw. R²-Br in die Dietherverbindungen der allgemeinen Formel (I.2) überführt. Bezüglich der Reste R¹ und R² wird auf die vorherigen Ausführungen Bezug genommen. Bevorzugt wird als Base ein Alkalihydroxid, insbesondere KOH, eingesetzt.

Zur Herstellung der erfindungsgemäßen Esterverbindungen der allgemeinen Formel (1.3) wird bevorzugt 2,5-Di-(hydroxymethyl)tetrahydrofuran bzw. 2,5-Di-(hydroxyethyl)-tetrahydrofuran mit wenigstens einem Säurehalogenid R¹-(C=O)X und, falls R¹ und R² unterschiedliche Bedeutung haben, mit wenigstens einem Säurehalogenid R²-(C=O)X, wobei X für Br oder Cl steht, in Gegenwart wenigstens eines tertiären Amins, zu den Verbindungen der Formel (I.3) umgesetzt.

Neben diesem Verfahren stehen dem Fachmann noch weitere geläufige Veresterungsmethoden zur Verfügung, wie zuvor im Falle der Veresterung von FDCS bzw. 2,5-Tetrahydrofurandicarbonsäure beschrieben.

Zur Herstellung der Esterverbindungen der allgemeinen Formel (I.3) können üblicherweise alle dem Fachmann geläufige Arten von tertiären Aminen verwendet werden. Beispiele für geeignete tertiäre Amine sind:
- aus der Gruppe der Trialkylamine: Trimethylamin, Triethylamin, Tri-n-propylamin, Diethylisopropylamin, Diisopropylethylamin und dergleichen;
- aus der Gruppe der N-Cycloalkyl-N,N-dialkylamine: Dimethylcyclohexylamin und Diethylcyclohexylamin;
- aus der Gruppe der N,N-Dialkylaniline: Dimethylanilin und Diethylanilin;
- aus der Gruppe der Pyridin- und Chinolinbasen: Pyridin, α-, β- und γ-Picolin, Chinolin und 4-(Dimethylamino)pyridin (DMAP).

Bevorzugte tertiäre Amine sind Trialkylamine und Pyridinbasen, insbesondere Triethylamin und 4-(Dimethylamino)pyridin (DMAP) sowie Gemische davon.

Die Veresterung kann bei Umgebungsdruck, bei vermindertem oder erhöhtem Druck erfolgen. Bevorzugt wird die Veresterung bei Umgebungsdruck durchgeführt.

Die Veresterung kann in Abwesenheit oder in Gegenwart eines organischen Lösungsmittels durchgeführt werden. Bevorzugt wird die Veresterung in Gegenwart eines inerten organischen Lösungsmittels, wie zuvor definiert, durchgeführt.

Die Veresterung wird üblicherweise in einem Temperaturbereich von 50 bis 200°C durchgeführt.

Die Veresterung kann in Abwesenheit oder in Gegenwart eines Inertgases erfolgen.

In einer bevorzugten Ausführungsform wird 2,5-Di-(hydroxymethyl)tetrahydrofuran mit einem Säurechlorid R¹-(C=O)Cl in Gegenwart von Triethylamin und/oder DMAP und eines inerten organischen Lösungsmittels zu Verbindungen der Formel (I.3) umgesetzt.

In den bevorzugten Ausführungsformen der erfindungsgemäßen Verfahren zur Herstellung von Verbindungen der allgemeinen Formel (I) werden für die Umesterung, Veresterung oder Alkylierung n-Butanol und/oder Isobutanol als Edukte verwendet.

Die für die bevorzugten Verfahren zur Herstellung von Verbindungen der allgemeinen Formel (I) als Ausgangsstoff benötigte Furan-2,5-dicarbonsäure (FDCS, CAS Nr. 3238-40-2) kann entweder kommerziell erworben oder nach literaturbekannten Synthesewegen hergestellt werden. So finden sich Möglichkeiten zur Synthese in der im Internet veröffentlichten Publikation von Lewkowski et al. mit dem Titel "Synthesis, Chemistry and Application of 5-hydroxymethylfurfural and its derivatives" (Lewkowski et al., ARKIVOC 2001 (i), Seiten 17-54, ISSN 1424-6376). Den meisten dieser Synthesen gemein ist eine säurekatalysierte Umsetzung von Kohlenhydraten, besonders Glucose, Fructose, bevorzugt Fructose zum 5-Hydroxymethylfurfural (5-HMF) welches durch verfahrenstechnische Operationen wie beispielsweise Zweiphasen-Fahrweise aus dem Reaktionsgemisch abgetrennt werden kann. Entsprechende Ergebnisse wurden beispielsweise von Leshkov et al. in Science 2006, Vol. 312, Seiten 1933-1937 und von Zhang et al. in Angewandte Chemie 2008, Vol. 120, Seiten 9485-9488 beschrieben. In einem weiteren Schritt kann die 5-HMF dann zu FDCS oxidiert werden, wie beispielsweise von Christensen in ChemSusChem 2007, Vol. 1, Seiten 75-78 zitiert.

2,5-Bis(hydroxymethyl)tetrahydrofuran (CAS Nr. 104-80-3) kann ebenfalls entweder kommerziell erworben oder synthetisiert werden. Die beschriebenen Synthesen erfolgen ausgehend von 5-HMF welches in zwei Schritten über 2,5-Bis(hydroxymethyl)furan (2,5-BHF) oder direkt zu 2,5-Di(hydroxymethyl)tetrahydrofuran reduziert werden kann (Lewkowski et al., ARKIVOC 2001 (i), Seiten 17-54, ISSN 1424-6376).

2,5-Bis(hydroxyethyl)tetrahydrofuran kann durch Reduktion des 2,5-furandiessigsäuremethylesters erhalten werden. 2,5-Furandiessigsäuremethylester kann über geeignete dem Fachmann geläufige Reaktionen aus 2,5-Bis(hydroxymethyl)furan (2,5-BHF) synthetisiert werden, wie beispielsweise analog dem von Rau et al. in Liebigs Ann. Chem., Vol. 1984 (8. 1984), Seiten 1504-1512, ISSN 0947-3440, beschriebenen Verfahren. Dabei wird aus 2,5-BHF durch Umsetzung mit Thionylchlorid 2,5-Bis(chlormethyl)furan dargestellt, welches durch Einwirkung von KCN in Benzol in Gegenwart von [18]Krone-6 zu 2,5-Bis(cyanomethyl)furan umgesetzt wird. Das 2,5-Bis(cyanomethyl)furan kann anschließend zur 2,5-furandiessigsäure verseift und mit Methanol zum Dimethylester verestert werden oder durch Alkoholyse mit Methanol direkt in den 2,5-Furandiessigsäuremethylester überführt werden (Pinner-Reaktion). Der 2,5-Furandiessigsäuremethylester kann dann entweder zuerst zum Tetrahydro-2,5-furandiessigsäuredimethylester hydriert (analog der Schritte b2) bzw. c1)) oder direkt zu 2,5-Bis(hydroxyethyl)tetrahydrofuran reduziert werden.

Die Darstellung des 2,5-furandiessigsäuremethylesters kann ebenfalls analog dem von Kern et al. in Liebigs Ann. Chem., Vol. 1985 (6. 1985), Seiten 1168-1174, ISSN 0947-3440, beschrieben Verfahren erfolgen.

### Weichmacher-Zusammensetzung

Die erfindungsgemäßen Verbindungen der allgemeinen Formel (I) zeichnen sich durch eine sehr gute Verträglichkeit mit einer Vielzahl verschiedener Weichmacher aus. Sie eignen sich speziell in Kombination mit anderen Weichmachern, die über noch verbesserungswürdige Geliereigenschaften verfügen, zur Verbesserung des Gelierverhaltens. So ermöglichen sie die Verringerung der zum Gelieren eines thermoplastischen Polymers erforderlichen Temperatur und/oder die Erhöhung der Geliergeschwindigkeit von Weichmacher-Zusammensetzungen.

Bei speziellen oder komplexen anwendungsbedingten Anforderungen, wie beispielsweise eine hohe Kälteelastizität, eine hohe Extraktions- oder Migrationsbeständigkeit oder sehr niedrige Weichmacherflüchtigkeit, kann es vorteilhaft sein, Weichmacher-Zusammensetzungen zur Weichmachung thermoplastischer Polymere zu verwenden. Dies gilt insbesondere für Weich-PVC-Anwendungen.

Aus diesem Grund betrifft die Erfindung auch Weichmacher-Zusammensetzungen, die wenigstens eine Verbindung der allgemeinen Formel (I) und wenigstens einen von den Verbindungen (I) verschiedenen Weichmacher enthalten.

Bezüglich geeigneter und bevorzugter Verbindungen der allgemeinen Formel (I) zur Herstellung von Weichmacher-Zusammensetzungen wird auf die zuvor beschriebenen geeigneten und bevorzugten Verbindungen der allgemeinen Formel (I) in vollem Umfang Bezug genommen. Bevorzugt enthalten die erfindungsgemäßen Weichmacher-Zusammensetzungen wenigstens eine Verbindung der allgemeinen Formel (I), worin R¹ und R² unabhängig voneinander für ein unverzweigtes oder verzweigtes C₄-Alkyl, insbesondere für n-Butyl oder Isobutyl steht. Eine speziell zur Herstellung von Weichmacher-Zusammensetzungen geeignete Verbindung der allgemeinen Formel (I) ist Di-(n-butyl)-2,5-tetrahydrofurandicarboxylat.

Der von den Verbindungen der allgemeinen Formel (I) verschiedene zusätzliche Weichmacher ist vorzugsweise ausgewählt unter Phthalsäuredialkylestern, Phthalsäurealkylaralkylestern, Terephthalsäuredialkylestern, Trimellithsäuretrialkylestern, Adipinsäuredialkylestern, Benzoesäurealkylestern, Dibenzoesäureestern von Glycolen, Hydroxybenzoesäureestern, Estern von gesättigten Mono- und Dicarbonsäuren, Estern von ungesättigten Dicarbonsäuren, Amiden und Estern von aromatischen Sulfonsäuren, Alkylsulfonsäureestern, Glycerinestern, Isosorbidestern, Phosphorsäureestern, Citronensäuretriestern, Alkylpyrrolidonderivaten, 2,5-Furandicarbonsäureestern, epoxidierten Pflanzenölen auf Basis von Triglyceriden und gesättigten oder ungesättigten Fettsäuren, Polyestern aus aliphatischen und aromatischen Polycarbonsäuren mit mehrwertigen Alkoholen.

Bevorzugte Phthalsäuredialkylester weisen unabhängig voneinander 4 bis 13 C-Atome, bevorzugt 8 bis 13 C-Atome, in den Alkylketten auf. Ein bevorzugter Phthalsäurealkylaralkylester ist beispielsweise Benzylbutylphthalat. Die Terephthalsäuredialkylester weisen bevorzugt unabhängig voneinander jeweils 4 bis 13 C-Atome, insbesondere 7 bis 11 C-Atome, in den Alkylketten auf. Bevorzugte Terephthalsäuredialkylester sind beispielsweise Di-(n-butyl)-terephthalsäuredialkylester, Di-(2-ethylhexyl)-terephthalsäuredialkylester, Di-(isononyl)-terephthalsäuredialkylester oder Di-(2-propylheptyl)-terephthalsäuredialkylester. Die Trimellithsäuretrialkylester weisen bevorzugt unabhängig voneinander jeweils 4 bis 13 C-Atome, insbesondere 7 bis 11 C-Atome, in den Alkylketten auf. Bei den Estern von gesättigten Mono- und Dicarbonsäuren handelt es sich bevorzugt um Ester der Essigsäure, Buttersäure, Valeriansäure, Bernsteinsäure, Adipinsäure, Sebacinsäure, Milchsäure, Äpfelsäure oder Weinsäure. Die Adipinsäuredialkylester weisen bevorzugt unabhängig voneinander jeweils 4 bis 13 C-Atome, insbesondere 6 bis 10 C-Atome, in den Alkylketten auf. Bei den Estern von ungesättigten Dicarbonsäuren handelt es sich bevorzugt um Ester der Maleinsäure und der Fumarsäure. Die Benzoesäurealkylester weisen bevorzugt unabhängig voneinander jeweils 7 bis 13 C-Atome, insbesondere 9 bis 13 C-Atome, in den Alkylketten auf. Bevorzugte Benzoesäurealkylester sind beispielsweise Isononylbenzoat, Isodecylbenzoat oder 2-Propylheptylbenzoat. Bevorzugte Dibenzoesäureester von Glycolen sind Diethylenglycoldibenzoat und Dibutylenglycol-dibenzoat. Bevorzugte Alkylsulfonsäureester weisen vorzugsweise einen Alkylrest mit 8 bis 22 C-Atomen auf. Dazu zählen beispielsweise der Phenyl- oder Cresylester der Pentadecylsulfonsäure. Bevorzugte Isosorbidester sind Isosorbiddiester, die bevorzugt unabhängig voneinander jeweils mit C₈ bis C₁₃-Carbonsäuren verestert sind. Bevorzugte Phosphorsäureester sind Tri-2-ethylhexylphosphat, Trioctylphosphat, Triphenylphosphat, Isodecyldiphenylphosphat, Bis-(2-ethylhexyl)phenyl-phosphat und 2-Ethylhexyldiphenylphosphat. In den Citronensäuretriestern kann die OH-Gruppe in freier oder carboxylierter Form, bevorzugt acetyliert, vorliegen. Die Alkylreste der Citronensäuretriester weisen bevorzugt unabhängig voneinander 4 bis 8 C-Atome, insbesondere 6 bis 8 C-Atome, auf. Bevorzugt sind Alkylpyrrolidonderivate mit Alkylresten von 4 bis 18 C-Atomen. Bevorzugte 2,5-Furandicarbonsäuredialkylester weisen unabhängig voneinander jeweils 4 bis 13 C-Atome, bevorzugt 8 bis 13 C-Atome, in den Alkylketten auf. Bei den epoxidierten Pflanzenölen handelt es sich beispielsweise bevorzugt um epoxidierte Fettsäuren aus epoxidertem Sojaöl, erhältlich unter dem Handelsnamen reFlex™ der Firma PolyOne, USA. Bei den Polyestern aus aliphatischen und aromatischen Polycarbonsäuren handelt es sich bevorzugt um Polyester der Adipinsäure mit mehrwertigen Alkoholen, insbesondere Dialkylenglycol-polyadipate mit 2 bis 6 Kohlenstoffatomen im Alkylenrest.

In allen zuvor genannten Fällen können die Alkylreste jeweils linear oder verzweigt und jeweils gleich oder verschieden sein. Auf die eingangs gemachten allgemeinen Ausführungen zu geeigneten und bevorzugten Alkylresten wird Bezug genommen.

In einer besonders bevorzugten Ausführungsform enthalten die erfindungsgemäßen Weichmacher-Zusammensetzungen wenigstens einen von den Verbindungen (I) verschiedenen Weichmacher, der ausgewählt ist unter Adipinsäuredialkylestern mit 4 bis 9 C-Atomen in der Seitenkette.

In einer weiteren besonders bevorzugten Ausführungsform enthalten die erfindungsgemäßen Weichmacher-Zusammensetzungen wenigstens einen C₅- bis C₁₁-Dialkylester der 2,5-Furandicarbonsäure. Besonders bevorzugt sind die C₇- bis C₁₀-Dialkylester der 2,5-Furandicarbonsäure.

Geeignete und bevorzugte Dialkylester der 2,5-Furandicarbonsäure sind in der WO 2012/113608 (C₅-Dialkylester), WO 2012/113609 (C₇-Dialkylester), WO 2011/023490 (C₉-Dialkylester) und WO 2011/023491 (C₁₀-Dialkylester) beschrieben. Die Dihexyl-, Di(2-ethylhexyl)- und Di(2-octyl)ester der 2,5-Furandicarbonsäure und ihre Herstellung werden von R. D. Sanderson et al. in J. Appl. Pol. Sci., 1994, Vol. 53, 1785-1793 beschrieben. Auf die Offenbarung dieser Dokumente wird hier in vollem Umfang Bezug genommen.

Besonders bevorzugte Dialkylester der 2,5-Furandicarbonsäure sind die in der WO 2011/023490 offenbarten isomeren Nonylester der 2,5-Furandicarbonsäure. Die isomeren Nonylreste leiten sich dabei vorzugsweise von einem Gemisch isomerer Nonanole ab, wie es in der WO 2011/023490, Seite 6, Zeile 32 bis Seite 10, Zeile 15 beschrieben ist.

### Formmassen

Ein weiterer Gegenstand der vorliegenden Erfindung betrifft eine Formmasse enthaltend wenigstens ein thermoplastisches Polymer und wenigstens eine Verbindung der allgemeinen Formel (I).

Als thermoplastische Polymere kommen alle thermoplastisch verarbeitbaren Polymeren in Frage. Insbesondere sind diese thermoplastischen Polymere ausgewählt unter:
- Homo- und Copolymere, die in einpolymerisierter Form wenigstens ein Monomer enthalten, das ausgewählt ist unter C₂-C₁₀ Monoolefinen, wie beispielsweise Ethylen oder Propylen, 1,3-Butadien, 2-Chlor-1,3-Butadien, Vinylalkohol und dessen C₂-C₁₀-Alkylestern, Vinylchlorid, Vinylidenchlorid, Vinylidenfluorid, Tetraf-Iuorethylen, Glycidylacrylat, Glycidylmethacrylat, Acrylaten und Methacrylaten mit Alkoholkomponenten von verzweigten und unverzweigten C₁-C₁₀-Alkoholen, Vinylaromaten wie beispielsweise Polystyrol, (Meth)acrylnitril, α,β-ethylenisch ungesättigten Mono- und Dicarbonsäuren, und Maleinsäureanhydrid;
- Homo- und Copolymere von Vinylacetalen;
- Polyvinylestern;
- Polycarbonaten (PC);
- Polyestern, wie Polyalkylenterephthalaten, Polyhydroxyalkanoaten (PHA), Polybutylensuccinaten (PBS), Polybutylensuccinatadipaten (PBSA);
- Polyethern;
- Polyetherketonen;
- thermoplastischen Polyurethanen (TPU);
- Polysulfiden;
- Polysulfonen;
und Mischungen davon.

Zu nennen sind beispielsweise Polyacrylate mit gleichen oder verschiedenen Alkoholresten aus der Gruppe der C₄-C₈-Alkohole, besonders des Butanols, Hexanols, Octanols und 2-Ethylhexanols, Polymethylmethacrylat (PMMA), Methylmethacrylat-Butylacrylat-Copolymere, Acrylnitril-Butadien-Styrol-Copolymere (ABS), Ethylen-Propylen-Copolymere, Ethylen-Propylen-Dien-Copolymere (EPDM), Polystyrol (PS), Styrol-Acrylnitril-Copolymere (SAN), Acrylnitril-Styrol-Acrylat (ASA), Styrol-Butadien-Methylmethacrylat-Copolymere (SBMMA), Styrol-Maleinsäureanhydrid-Copolymere, Styrol-Methacrylsäure-Copolymere (SMA), Polyoxymethylen (POM), Polyvinylalkohol (PVAL), Polyvinylacetat (PVA), Polyvinylbutyral (PVB), Polycaprolacton (PCL), Polyhydroxybuttersäure (PHB), Polyhydroxyvaleriansäure (PHV), Polymilchsäure (PLA), Ethylcellulose (EC), Celluloseacetat (CA), Cellulosepropionat (CP) oder CelluloseAcetat/Butyrat (CAB).

Bevorzugt handelt es sich bei dem in der erfindungsgemäßen Formmasse enthaltenen wenigstens einem thermoplastischen Polymer um Polyvinylchlorid (PVC), Polyvinylbutyral (PVB), Homo- und Copolymere von Vinylacetat, Homo- und Copolymere von Styrol, Polyacrylate, thermoplastische Polyurethane (TPU) oder Polysulfide.

Ein weiterer Gegenstand der vorliegenden Erfindung betrifft Formmassen enthaltend wenigstens ein Elastomer und wenigstens eine Verbindung der allgemeinen Formel (I).

Bevorzugt handelt es bei dem in den erfindungsgemäßen Formmassen enthaltenen Elastomer um wenigstens einen natürlichen Kautschuk (NR), wenigstens einen auf synthetischem Wege hergestellten Kautschuk oder Mischungen davon. Bevorzugte auf synthetischem Wege hergestellte Kautschuke sind beispielsweise PolyisoprenKautschuk (IR), Styrol-Butadien-Kautschuk (SBR), Butadien-Kautschuk (BR), Nitril-Butadien-Kautschuk (NBR) oder Chloropren-Kautschuk (CR).

Bevorzugt sind Kautschuke oder Kautschuk-Mischungen, welche sich mit Schwefel vulkanisieren lassen.

Im Rahmen der Erfindung liegt der Gehalt (Gew.-%) an Elastomer in den Formmassen bei 20 bis 99%, bevorzugt 45 bis 95%, besonders bevorzugt bei 50 bis 90% und insbesondere bei 55 bis 85%.

In der erfindungsgemäßen Formmasse können neben wenigstens einem Elastomer und wenigstens einem Tetrahydrofuranderivat der allgemeinen Formel (I), wenigstens einen zu den Verbindungen (I) verschiedenen Weichmacher enthalten sein.

Geeignete zu den Verbindungen (I) verschiedenen Weichmacher sind solche wie bereits oben definiert.

Im Rahmen der Erfindung können die Formmassen, welche wenigstens ein Elastomer enthalten, zusätzlich zu den vorstehenden Bestandteilen andere geeignete Zusatzstoffe enthalten. Beispielsweise können verstärkende Füllstoffe, wie Ruß oder Siliciumdioxid, weitere Füllstoffe, ein Methylendonor, wie Hexamethylentetramin (HMT), ein Methylenakzeptor, wie mit Cardanol (aus Cashew-Nüssen) modifizierte Phenolharze, ein Vulkanisier- oder Vernetzungsmittel, ein Vulkanisier- oder Vernetzungsbeschleuniger, Aktivatoren, verschiedene Typen von Öl, Alterungsschutzmittel und andere verschiedene Zusatzstoffe, die beispielsweise in Reifen- und anderen Kautschukmassen eingemischt werden, enthalten sein.

Im Speziellen handelt es sich bei dem in der erfindungsgemäßen Formmasse enthaltenen wenigstens einem thermoplastischen Polymer um Polyvinylchlorid (PVC).

Polyvinylchlorid wird durch Homopolymerisation von Vinylchlorid erhalten. Das erfindungsgemäß verwendete Polyvinylchlorid (PVC) kann beispielsweise durch Suspensionspolymerisation, Mikrosuspensionspolymerisation, Emulsionspolymerisation oder Massenpolymerisation hergestellt werden. Die Herstellung von PVC durch Polymerisation von Vinylchlorid sowie Herstellung und Zusammensetzung von weichgemachtem PVC sind beispielsweise beschrieben in "Becker/Braun, Kunststoff-Handbuch, Band 2/1 : Polyvinylchlorid", 2. Auflage, Carl Hanser Verlag, München.

Der K-Wert, der die Molmasse des PVC charakterisiert und nach DIN 53726 bestimmt wird, liegt für das erfindungsgemäß weichgemachte PVC meist zwischen 57 und 90, bevorzugt zwischen 61 und 85, insbesondere zwischen 64 und 75.

Im Rahmen der Erfindung liegt der Gehalt an PVC der Gemische bei 20 bis 99 Gew.-%, bevorzugt bei 45 bis 95 Gew.-%, besonders bevorzugt bei 50 bis 90 Gew.-% und insbesondere bei 55 bis 85 Gew.-%.

In der erfindungsgemäßen Formmasse können neben wenigstens einem thermoplastischen Polymer und wenigstens einem Tetrahydrofuranderivat der allgemeinen Formel (I), wenigstens einen zu den Verbindungen (I) verschiedenen Weichmacher enthalten sein.

Der Anteil des zusätzlichen wenigstens einen zu den Verbindungen (I) verschiedenen Weichmachers in der erfindungsgemäßen Formmasse liegt bei 10 bis 90 Gew.-%, bevorzugt bei 20 bis 85 Gew.-% und besonders bevorzugt bei 50 bis 80 Gew.-%, bezogen auf die in der Formmasse enthaltenen Gesamtweichmachermenge.

Geeignete zu den Verbindungen (I) verschiedenen Weichmacher sind solche wie bereits oben definiert.

Besonders bevorzugt ist der in der erfindungsgemäßen Formmasse enthaltene, mindestens eine zusätzliche Weichmacher ausgewählt unter Adipinsäuredialkylestern mit 4 bis 9 C-Atomen in der Seitenkette und 2,5-Furandicarbonsäureester mit 4 bis 10 C-Atomen in der Seitenkette, wobei die Estergruppen entweder die gleiche oder eine voneinander verschiedene Anzahl an Kohlenstoffatomen aufweisen können.

Je nachdem welches thermoplastische Polymer oder thermoplastische Polymergemisch in der Formmasse enthalten ist, werden unterschiedliche Mengen Weichmacher eingesetzt. In der Regel beträgt der Gesamtweichmachergehalt in der Formmasse 0,5 bis 300 phr (parts per hundred resin = Gewichtsteile pro hundert Gewichtsteile Polymer), bevorzugt 0,5 bis 130 phr, besonders bevorzugt 1 bis 35 phr.

Handelt es sich bei dem thermoplastischen Polymer in den erfindungsgemäßen Frommassen um Polyvinylchlorid und wird als Weichmacher ausschließlich wenigstens einer der erfindungsgemäßen (C₇-C₁₂)-Dialkylester der Tetrahydrofurandicarbonsäure verwendet, beträgt der Gesamtweichmachergehalt in der Formmasse 5 bis 300 phr, bevorzugt 10 bis 100 phr und besonders bevorzugt 30 bis 70 phr.

Handelt es sich bei dem thermoplastischen Polymer in den erfindungsgemäßen Formmassen um Polyvinylchlorid und werden Weichmachergemische, enthaltend wenigstens eine Verbindung der allgemeinen Formel (I) und wenigstens einen zu den Verbindungen (I) verschiedenen Weichmacher, verwendet, beträgt der Gesamtweichmachergehalt in der Formmasse 1 bis 400 phr, bevorzugt 5 bis 130 phr, besonders bevorzugt 10 bis 100 phr und insbesondere 15 bis 85 phr.

Handelt es sich bei dem Polymer in den erfindungsgemäßen Formmassen um Kautschuke, beträgt der Gesamtweichmachergehalt in der Formmasse 1 bis 60 phr, bevorzugt 1 bis 40 phr, besonders bevorzugt 2 bis 30 phr.

### Zusatzstoffe Formmasse

Im Rahmen der Erfindung können die Formmassen, enthaltend wenigstens ein thermoplastisches Polymer, andere geeignete Zusatzstoffe enthalten. Beispielsweise können Stabilisatoren, Gleitmittel, Füllstoffe, Pigmente, Flamminhibitoren, Lichtstabilisatoren, Treibmittel, polymere Verarbeitungshilfsmittel, Schlagzähverbesserer, optische Aufheller, Antistatika oder Biostabilisatoren enthalten sein.

Im Folgenden werden einige geeignete Zusatzstoffe näher beschrieben. Die aufgeführten Beispiele stellen jedoch keine Einschränkung der erfindungsgemäßen Formmassen dar, sondern dienen lediglich der Erläuterung. Alle Angaben zum Gehalt sind in Gew.-%-Angaben bezogen auf die gesamte Formmasse.

Als Stabilisatoren kommen alle üblichen PVC-Stabilisatoren in fester und flüssiger Form in Betracht, beispielsweise übliche Ca/Zn-, Ba/Zn-, Pb- oder Sn-Stabilisatoren sowie auch säurebindende Schichtsilikate wie Hydrotalcit.

Die erfindungsgemäßen Formmassen können einen Gehalt an Stabilisatoren von 0,05 bis 7%, bevorzugt 0,1 bis 5%, besonders bevorzugt von 0,2 bis 4% und insbesondere von 0,5 bis 3% aufweisen.

Gleitmittel sollten zwischen den PVC-Pastillen wirksam werden und Reibungskräften beim Mischen, Plastifizieren und Verformen entgegenwirken.

Als Gleitmittel können die erfindungsgemäßen Formmassen alle die für die Verarbeitung von Kunststoffen üblichen Gleitmittel enthalten. Beispielsweise kommen in Betracht Kohlenwasserstoffe, wie Öle, Paraffine und PE-Wachse, Fettalkohole mit 6 bis 20 Kohlenstoffatomen, Ketone, Carbonsäuren, wie Fettsäuren und Montansäure, oxidiertes PE-Wachs, Metallsalze von Carbonsäuren, Carbonsäureamide sowie Carbonsäureester, beispielsweise mit den Alkoholen Ethanol, Fettalkoholen, Glycerin, Ethandiol, Pentaerythrit und langkettigen Carbonsäuren als Säurekomponente.

Die erfindungsgemäßen Formmassen können einen Gehalt an Gleitmittel von 0,01 bis 10%, bevorzugt 0,05 bis 5%, besonders bevorzugt von 0,1 bis 3% und insbesondere von 0,2 bis 2% aufweisen.

Füllstoffe beeinflussen vor allem die Druck-, Zug-, und Biegefestigkeit sowie die Härte und Wärmeformbeständigkeit von weichgemachtem PVC in positiver Weise.

Im Rahmen der Erfindung können die Formmassen auch Füllstoffe wie beispielsweise Ruß und andere organische Füllstoffe, wie natürliche Calciumcarbonate, beispielsweise Kreide, Kalkstein und Marmor, synthetische Calciumcarbonate, Dolomit, Silikate, Kieselsäure, Sand, Diatomeenerde, Aluminiumsilikate, wie Kaolin, Glimmer und Feldspat enthalten. Vorzugsweise werden als Füllstoffe, Calciumcarbonate, Kreide, Dolomit, Kaolin, Silikate, Talkum oder Ruß eingesetzt.

Die erfindungsgemäßen Formmassen können einen Gehalt an Füllstoffen von 0,01 bis 80%, bevorzugt 0,1 bis 60%, besonders bevorzugt von 0,5 bis 50% und insbesondere von 1 bis 40% aufweisen.

Die erfindungsgemäßen Formmassen können auch Pigmente enthalten, um das erhaltenen Produkt an unterschiedliche Einsatzmöglichkeiten anzupassen.

Im Rahmen der vorliegenden Erfindung können sowohl anorganische Pigmente als auch organische Pigmente eingesetzt werden. Als anorganische Pigmente können beispielsweise Cadmium-Pigmente, wie CdS, Kobalt-Pigmente, wie CoO/Al₂O₃, und Chrom-Pigmente, beispielsweise Cr₂O₃, verwendet werden. Als organische Pigmente kommen beispielsweise Monoazopigmente, kondensierte Azopigmente, Azomethinpigmente, Anthrachinonpigmente, Chinacridone, Phthalocyaninpigmente, Dioxazinpigmente und Anilinpigmente in Betracht.

Die erfindungsgemäßen Formmassen können einen Gehalt an Pigmenten von 0,01 bis 10%, bevorzugt 0,05 bis 5%, besonders bevorzugt von 0,1 bis 3% und insbesondere von 0.5 bis 2% aufweisen.

Um die Entflammbarkeit zu vermindern und die Rauchentwicklung beim Verbrennen zu verringern, können die erfindungsgemäßen Formmassen auch Flamminhibitoren enthalten.

Als Flamminhibitoren können beispielsweise Antimontrioxid, Phosphatester, Chlorparaffin, Aluminiumhydroxid, Borverbindungen, Molybdäntrioxid, Ferrocen, Calciumcarbonat oder Magnesiumcarbonat verwendet werden.

Die erfindungsgemäßen Formmassen können einen Gehalt an Flamminhibitoren von 0,01 bis 10%, bevorzugt 0,1 bis 8%, besonders bevorzugt von 0,2 bis 5% und insbesondere von 0,5 bis 2% aufweisen.

Um aus den erfindungsgemäßen Formmassen hergestellte Artikel vor einer Schädigung im Oberflächenbereich durch den Einfluss von Licht zu schützen, können die Formmassen auch Lichtstabilisatoren enthalten.

Es können im Rahmen der vorliegenden Erfindung beispielsweise Hydroxybenzophenone oder Hydroxyphenylbenzotriazole eingesetzt werden.

Die erfindungsgemäßen Formmassen können einen Gehalt an Lichtstabilisatoren von 0,01 bis 7%, bevorzugt 0,1 bis 5%, besonders bevorzugt von 0,2 bis 4% und insbesondere von 0,5 bis 3% aufweisen.

### Plastisolanwendungen

Wie bereits dargelegt eignen sich die erfindungsgemäßen Verbindungen aufgrund ihrer guten Geliereigenschaften insbesondere zur Herstellung von Plastisolen.

Plastisole können aus verschiedenen Kunststoffen hergestellt werden. Bei den erfindungsgemäßen Plastisolen handelt es sich in einer bevorzugten Ausführungsform um ein PVC-Plastisol.

Die erfindungsgemäßen Plastisole können neben wenigstens einem Kunststoff und wenigstens einem Tetrahydrofuranderivat der allgemeinen Formel (I), gegebenenfalls wenigstens einen zu den Verbindungen (I) verschiedenen Weichmacher enthalten.

Der Anteil des zusätzlichen wenigstens einen zu den Verbindungen (I) verschiedenen Weichmachers im Plastisol liegt bei 10 bis 90 Gew.-%, bevorzugt bei 20 bis 85 Gew.-% und besonders bevorzugt bei 50 bis 80 Gew.-% bezogen auf die im Plastisol enthaltenen Gesamtweichmachermenge.

Bei PVC-Plastisolen, welche als Weichmacher ausschließlich wenigstens einen der erfindungsgemäßen (C₇-C₁₂)-Dialkylester der Tetrahydrofurandicarbonsäure enthalten, beträgt der Gesamtweichmacheranteil üblicherweise 5 bis 300 phr, bevorzugt 10 bis 100 phr.

Bei PVC-Plastisolen, welche als Weichmacher wenigstens eine Verbindung der allgemeinen Formel (I) und wenigstens einen zu den Verbindungen (I) verschiedenen Weichmacher enthalten, beträgt der Gesamtweichmacheranteil üblicherweise 5 bis 400 phr, bevorzugt 50 bis 200 phr.

Plastisole werden üblicherweise bei Umgebungstemperatur durch verschiedene Verfahren, wie Streichverfahren, Gießverfahren, wie das Schalengieß- oder Rotationsgießverfahren, Tauchverfahren, Spritzverfahren und dergleichen in die Form des fertigen Produkts gebracht. Anschließend erfolgt durch Erwärmung die Gelierung, wobei nach Abkühlung ein homogenes, mehr oder weniger flexibles Produkt erhalten wird.

PVC-Plastisole eignen sich insbesondere zur Herstellung für PVC-Folien, für die Herstellung von nahtlosen Hohlkörpern, Handschuhen und zur Anwendung im Textilbereich, wie z. B. für Textilbeschichtungen.

### Anwendungen Formmasse

Die erfindungsgemäße Formmasse wird bevorzugt für die Herstellung von Formkörpern und Folien verwendet. Dazu gehören insbesondere Gehäuse von Elektrogeräten, wie beispielsweise Küchengeräten und Computergehäuse; Werkzeuge; Apparate; Rohrleitungen; Kabel; Schläuche, wie beispielsweise Kunststoffschläuche, Wasser- und Bewässerungsschläuche, Industrie-Gummischläuche oder Chemieschläuche; Draht-Ummantelungen; Fensterprofile; Komponenten für den Fahrzeugbau, wie beispielsweise Karosseriebestandteile, Vibrationsdämpfer für Motoren; Reifen; Möbel, wie beispielsweise Stühle, Tische oder Regale; Schaumstoff für Polster und Matratzen; Planen, wie beispielsweise LKW-Planen oder Zeltplanen; Dichtungen; Verbundfolien, wie Folien für Verbundsicherheitsglas, insbesondere für Fahrzeug- und Fensterscheiben; Schallplatten; Kunstleder; Verpackungsbehälter; Klebebandfolien oder Beschichtungen.

Daneben eignet sich die erfindungsgemäße Formmasse zusätzlich für die Herstellung von Formkörpern und Folien, die direkt mit Menschen oder Nahrungsmitteln in Kontakt kommen. Dabei handelt es sich vorwiegend um Medizinprodukte, Hygieneprodukte, Lebensmittelverpackungen, Produkte für den Innenraumbereich, Spielzeug und Kinderpflegeartikel, Sport- und Freizeitprodukte, Bekleidung sowie Fasern für Gewebe und dergleichen.

Bei den Medizinprodukten, die aus der erfindungsgemäßen Formmasse hergestellt werden können, handelt es sich beispielsweise um Schläuche für enterale Ernährung und Hämodialyse, Beatmungsschläuche, Infusionsschläuche, Infusionsbeutel, Blutbeutel, Katheter, Trachealtuben, Einmalspritzen, Handschuhe oder Atemmasken.

Bei den Lebensmittelverpackungen, die aus der erfindungsgemäßen Formmasse hergestellt werden können, handelt es sich beispielsweise um Frischhaltefolien, Lebensmittelschläuche, Trinkwasserschläuche, Behälter zur Aufbewahrung oder zum Einfrieren von Lebensmitteln, Deckeldichtungen, Verschlusskappen, Kronkorken oder künstliche Weinkorken.

Bei den Produkte für den Innenraumbereich, die aus der erfindungsgemäßen Formmasse hergestellt werden können, handelt es sich beispielsweise um Bodenbeläge, welche homogen oder aus mehreren Schichten, bestehend aus mindestens einer geschäumten Schicht, aufgebaut sein können, wie beispielsweise Fußbodenbeläge, Sportböden oder Luxury Vinyl Tiles (LVT), Kunstleder, Wandbeläge oder geschäumte oder nicht geschäumte Tapeten in Gebäuden oder um Verkleidungen oder Konsolenabdeckungen in Fahrzeugen.

Bei den Spielzeugen und Kinderpflegeartikeln, die aus der erfindungsgemäßen Formmasse hergestellt werden können, handelt es sich beispielsweise um Puppen, aufblasbares Spielzeug wie Bälle, Spielfiguren, Knete, Schwimmhilfen, Kinderwagen-Abdeckhauben, Wickelauflagen, Wärmflaschen, Beißringe oder Fläschchen.

Bei den Sport und Freizeitprodukte, die aus der erfindungsgemäßen Formmasse hergestellt werden können, handelt es sich beispielsweise um Gymnastikbälle, Übungsmatten, Sitzkissen, Massagebälle und -rollen, Schuhe bzw. Schuhsolen, Bälle, Luftmatratzen oder Trinkflaschen.

Bei der Bekleidung, die aus den erfindungsgemäßen Formmassen hergestellt werden können, handelt es sich beispielsweise um Latex-Kleidung, Schutzbekleidung, Regenjacken oder Gummistiefel.

### Nicht-PVC-Anwendungen:

Daneben beinhaltet die vorliegende Erfindung die Verwendung der erfindungsgemäßen Verbindungen als oder/und in Hilfsmitteln ausgewählt unter: Kalandrierhilfsmitteln; Rheologiehilfsmitteln; Oberflächenaktive Zusammensetzungen wie Fließ-, Filmbildehilfen, Entschäumern, Schaumverhütern, Benetzungsmitteln, Koaleszenzmitteln und Emulgatoren; Schmierstoffen, wie Schmierölen, Schmierfetten und Schmierpasten; Quenchern für chemische Reaktionen; Phlegmatisierungsmitteln; pharmazeutischen Produkten; Weichmachern in Klebstoffen; Schlagzähmodifizierern und Stellmitteln.

Die Erfindung wird anhand der im Folgenden beschriebenen Figuren und der Beispiele näher erläutert. Dabei sollen die Figuren und Beispiele nicht als einschränkend für die Erfindung verstanden werden.

In den nachfolgenden Beispielen und Figuren werden folgende Abkürzungen verwendet:
2,5-FDCS für 2,5-Furandicarbonsäure,
2,5-THFDCS für 2,5-Tetrahydrofurandicarbonsäure,
DMAP für 4-Dimethylaminopyridin,
TBME für tert-Butylmethylether,
THF für Tetrahydrofuran,
phr für Gewichtsteile pro 100 Gewichtsteile Polymer.

### FIGURENBESCHREIBUNG

Figur 1:

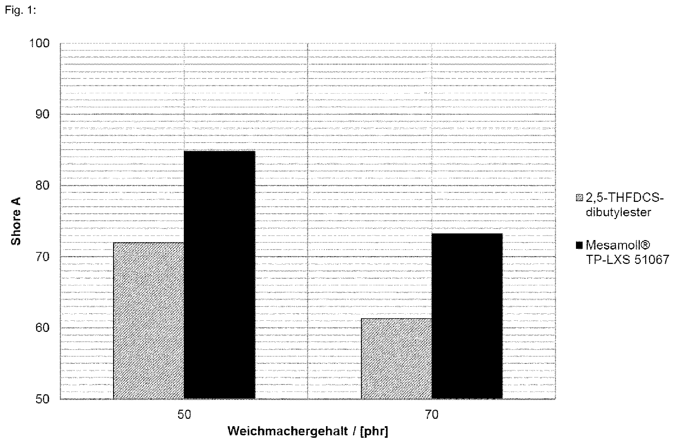

   Figur 1 zeigt in Form eines Balkendiagramms die Shore-Härte A von Weich-PVC-Probekörpern, welche die Weichmacher 2,5-THFDCS-dibutylester (weiß schraffiert) und als Vergleich den kommerziell erhältlichen Weichmacher Mesamoll® TP-LXS 51067 (schwarz) in unterschiedlichen Mengen enthalten. Aufgetragen ist die Shore-Härte A gegen den Weichmachergehalt der Weich-PVC-Probekörper (angegeben in phr). Die Messwerte wurden immer nach einer Zeit von 15 Sekunden ermittelt.
Figur 2:

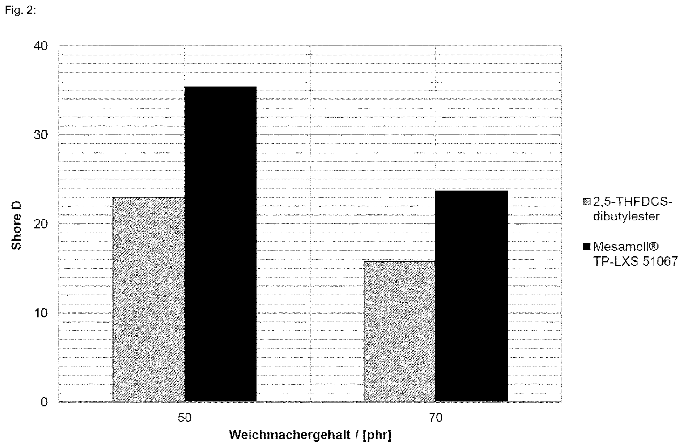

   Figur 2 zeigt in Form eines Balkendiagramms die Shore-Härte D von Weich-PVC-Probekörpern, welche den erfindungsgemäßen Weichmacher 2,5-THFDCS-dibutylester (weiß schraffiert) und als Vergleich den kommerziell erhältlichen Weichmacher Mesamoll® TP-LXS 51067 (schwarz) in 50 bzw. 70 phr enthalten. Aufgetragen ist die Shore-Härte D gegen den Weichmachergehalt der Weich-PVC-Probekörper (angegeben in phr). Die Messwerte wurden immer nach einer Zeit von 15 Sekunden ermittelt.
Figur 3:

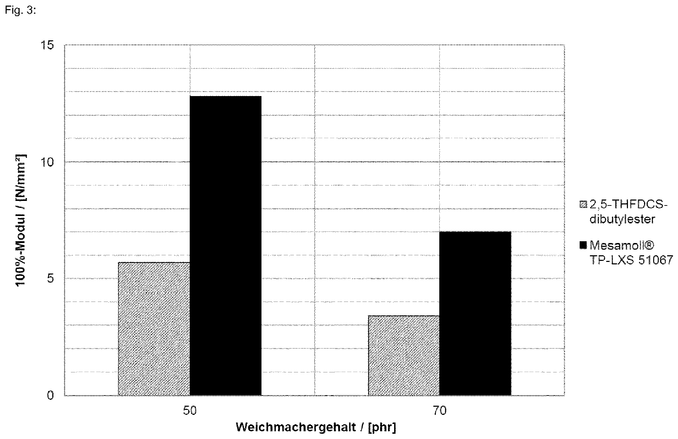

   Figur 3 zeigt in Form eines Balkendiagramms das 100%-Modul von Weich-PVC-Probekörpern, welche den erfindungsgemäßen Weichmacher 2,5-THFDCS-dibutylester (weiß schraffiert) und als Vergleich den kommerziell erhältlichen Weichmacher Mesamoll® TP-LXS 51067 (schwarz) in 50 bzw. 70 phr enthalten. Aufgetragen ist das 100% Modul gegen den Weichmachergehalt der Weich-PVC-Probekörper (angegeben in phr).
Figur 4:

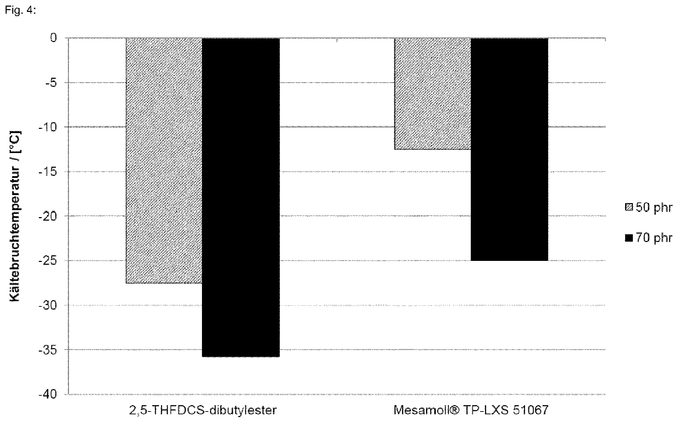

   Figur 4 zeigt in Form eines Balkendiagramms die Kältebruchtemperatur von Weich-PVC-Folien, welche den erfindungsgemäßen Weichmacher 2,5-THFDCS-dibutylester und als Vergleich den kommerziell erhältlichen Weichmacher Mesamoll® TP-LXS 51067 enthalten. Aufgetragen ist die Kältebruchtemperatur in °C für Weich-PVC-Folien mit einem Weichmachergehalt von jeweils 50 bzw. 70 phr.
Figur 5:

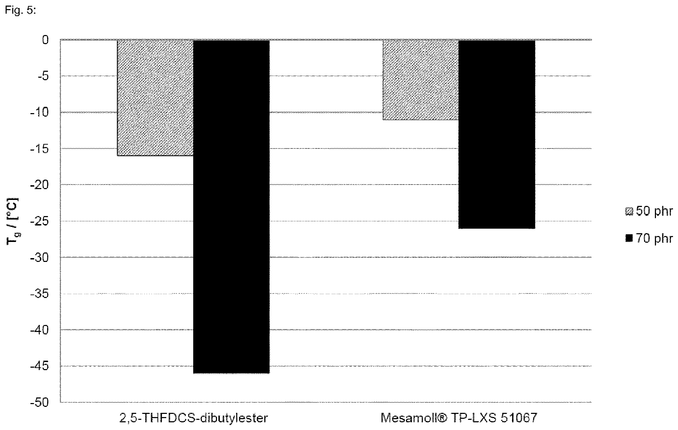

   Figur 5 zeigt in Form eines Balkendiagramms die Glasübergangstemperatur (T_{g}) von Weich-PVC-Folien, welche den erfindungsgemäßen Weichmacher 2,5-THFDCS-dibutylester und als Vergleich den kommerziell erhältlichen Weichmacher Mesamoll® TP-LXS 51067 enthalten. Aufgetragen ist die Glasübergangstemperatur (T_{g}) in °C für Weich-PVC-Folien mit einem Weichmachergehalt von jeweils 50 bzw. 70 phr.

### BEISPIELE

### I) Herstellungsbeispiele:

### Beispiel 1

### Synthese von Di-(n-butyl)-2,5-tetrahydrofurandicarboxylat aus Dimethyl-2,5-furandicarboxylat durch Umesterung und Hydrierung

### Beispiel 1.1:

### Herstellung von Dimethyl-2,5-furandicarboxylat (= Schritt a)

3,30 kg Methanol wurden zusammen mit 0,10 kg konzentrierter Schwefelsäure in einem 10 L Glasreaktor, ausgestattet mit Heizmantel, Rückflusskühler und mechanischem Rührer, vorgelegt. Dazu wurden unter heftigem Rühren 1,6 kg 2,5-Furandicarbonsäure (2,5-FDCS) langsam zugegeben. Die sich bildende dichte weiße Suspension wurde anschließend auf 70°C (Rückfluss) erhitzt. Der Reaktionsverlauf wurde mittels HPLC-Analyse verfolgt wobei man nach etwa 20 h eine klare Lösung unter vollständigem Umsatz der 2,5-FDCS erhielt. Daraufhin wurde die Reaktionsmischung auf 65°C abgekühlt und mit gesättigter NaHCO₃-Lösung und festem NaHCO₃ neutralisiert (pH 7). Während der Neutralisation bildete sich erneut eine dichte weiße Suspension, die auf 10°C abgekühlt, für weitere 0,5 h gerührt wurde und anschließend über eine P2 gesinterte Glasfritte abfiltriert wurde. Der Filterkuchen wurde dreimal mit 1 L kaltem Wasser gewaschen, worauf ungefähr 2 kg nasser Feststoff erhalten wurde. Zur Reinigung und Umkristallisation wurde der nasse Feststoff in einem 10 L Glasreaktor, ausgestattet mit Heizmantel, Rückflusskühler und mechanischem Rührer, zu 6,00 kg 2-Butanon zugegeben. Die Suspension wurde auf 70°C erhitzt worauf eine klare Lösung erhalten wurde. Anschließend wurde 1,00 kg Wasser hinzugegeben was zur Bildung einer braun-orangen wässrigen Phase führte. Gegebenenfalls war die Zugabe von 900 mL gesättigter Kochsalzlösung nötig, um eine Phasentrennung zu erreichen. Die wässrige Phase wurde entfernt und die organische Phase ohne Rühren auf 20°C abgekühlt, worauf die Kristallisation des Produktes einsetzte (üblicherweise bei etwa 35°C). Die kristalline Suspension wurde dann auf 0°C abgekühlt und über Nacht gerührt. Anschließend wurde die Suspension über eine P2 gesinterte Glasfritte abfiltriert und der Filterkuchen mit 1 L kaltem Methanol gewaschen. Der feste Rückstand wurde unter Vakuum bei Raumtemperatur getrocknet. Das gewünschte Dimethyl-2,5-furandicarboxylat wurde in einer Ausbeute von 50-60% und einer Reinheit von >99% erhalten. Die Identität und Reinheit des finalen Produktes wurde mittels NMR und HPLC ermittelt (HPLC Trennsäule: Varian Polaris 3µ C18-A, 150 x 4,6 mm).

### Beispiel 1.2:

### Katalytische Hydrierung (= Schritt b2)

Eine 20 gew.%-ige Lösung von Dimethyl-2,5-furandicarboxylat in THF wurde in einen stickstoffgefüllten 2,5 L Hastelloy-C Autoklaven der Firma Parr Instrument, bestückt mit einem mechanischen Rührer mit Magnetkupplung, Thermokupplung, Probeentnahmerohr und Stromstörer, gegeben. Anschließend wurden 120 g eines heterogenen Pd/Pt-Katalysators (0,4 Gew.-% Pd / 0,4 Gew.-% Pt auf ZrO₂, hergestellt analog zu DE4429014, Beispiel 6) zugegeben und die Stickstoffatmosphäre, durch dreimaliges Füllen und Belüften des Autoklaven mit Wasserstoff, gegen eine Wasserstoffatmosphäre ausgetauscht. Der finale Wasserstoffdruck wurde auf 200 bar erhöht und der Autoklav auf 180°C erhitzt. Der Reaktionsverlauf wurde mittels GC-Analyse verfolgt. Nach vollständigem Umsatz (üblicherweise nach 40 bis 60 Stunden) wurde der Autoklav abgekühlt, belüftet und der Inhalt abfiltriert, um den festen Katalysator zu entfernen. Das im Filtrat befindliche Lösungsmittel wurde anschließend unter reduziertem Druck destillativ entfernt, das zurückgebliebene Rohprodukt in 300 mL tert-Butylmethylether verdünnt und in einen Scheidetrichter überführt. Die organische Phase wurde zweimal mit gesättigter NaHCO₃-Lösung und einmal mit gesättigter Kochsalzlösung gewaschen. Anschließend wurden das Lösungsmittel und andere leichtflüchtige Bestandteile unter reduziertem Druck destillativ entfernt. Das Rohprodukt wurde mittels fraktionierter Destillation gereinigt, worauf Dimethyl-2,5-tetrahydrofurandicarboxylat als farblose bis leicht braune, viskose Flüssigkeit erhalten wurde. Das gewünschte Dimethyl-2,5-tetrahydrofurandicarboxylat konnte dabei in einer Ausbeute von 57% und einer Reinheit von 98,2% erhalten werden. Die Identität und Reinheit des Endprodukts wurde mittels NMR- und GC-MS-Analyse ermittelt (GC-Trennsäule: Agilent J&W DB-5, 30 m x 0,32 mm x 1,0 µm).

### Beispiel 1.3:

### Umesterung von Dimethyl-2,5-tetrahydrofurandicarboxylat (= Schritt c2)

In einem 2 L Rundhalskolben, ausgestattet mit einem Tropftrichter mit Druckausgleich, wurden 204 g (1,08 mol, 1,0 Äquivalente) Dimethyl-2,5-tetrahydrofurandicarboxylat in 200 g n-Heptan gelöst und 325 g (4,38 mol, 4,0 Äquivalente) n-Butanol sowie ein gemischter Titan(IV)-Propoxid / Butoxid-Komplex (3 mol-% Titan) zugegeben. Die Mischung wurde unter Rühren 22 Stunden bis zum Rückfluss erhitzt (100-126°C). Der Reaktionsverlauf wurde mittels GC-Analyse verfolgt. Nach vollständigem Umsatz wurde die Reaktionsmischung auf Raumtemperatur abgekühlt, filtriert und das Titan(IV)-Alkoxid durch die Zugabe von 100 mL Wasser hydrolysiert. Die zweiphasige Mischung wurde in einen Scheidetrichter überführt, die wässrige Phase entfernt und die organische Phase einmal mit gesättigter Kochsalzlösung gewaschen. Anschließend wurde das Lösungsmittel und andere leichtflüchtige Bestandteile unter reduziertem Druck destillativ entfernt. Das Rohprodukt wurde mittels fraktionierter Destillation gereinigt, worauf Di-(n-Butyl)-2,5-tetrahydrofurandicarboxylat als klare farblose Flüssigkeit in einer Ausbeute von 72% und einer Reinheit von 98,3% erhalten wurde. Die Identität und Reinheit des Endprodukts wurde mittels NMR- und GC-MS-Analyse ermittelt (GC-Trennsäule: Agilent J&W DB-5, 30 m x 0,32 mm x 1,0 µm).

### Beispiel 2

### Synthese von Di-(n-butyl)-2,5-tetrahydrofurandicarboxylat durch direkte Veresterung und Hydrierung

### Beispiel 2.1:

### Herstellung von Di-(n-butyl)-2,5-furandicarboxylat (= Schritt b1)

In einem 2 L Rundhalskolben, ausgestattet mit einem Dean-Stark Wasserabscheider und einem Tropftrichter mit Druckausgleich, wurden 445 g (6,00 mol, 4,0 Äquivalente) n-Butanol in 500 g Toluol vorgelegt. Die Mischung wurde unter Rühren bis zum Rückfluss erhitzt und 234 g (1,50 mol, 1,0 Äquivalente) 2,5-Furandicarbonsäure gefolgt von 11,5 g (0,12 mol, 8 mol-%) 99,9 %-iger Schwefelsäure in 3 bis 4 Portionen zugegeben wann immer sich die Umsetzung verlangsamte. Der Reaktionsverlauf wurde anhand der Menge an abgeschiedenem Wasser in der Dean-Stark-Apparatur verfolgt. Nach vollständigem Umsatz wurde dem Reaktionsgemisch eine Probe entnommen und mittels GC analysiert. Die Reaktionsmischung wurde auf Raumtemperatur abgekühlt, in einen Scheidetrichter überführt und zweimal mit gesättigter NaHCO₃-Lösung gewaschen. Die organische Phase wurde mit gesättigter Kochsalzlösung gewaschen, mit wasserfreiem Na₂SO₄ getrocknet und das Lösungsmittel unter reduziertem Druck entfernt. Das Rohprodukt wurde mittels fraktionierter Destillation gereinigt. Das gewünschte Di-(n-butyl)-2,5-furandicarboxylat konnte dabei in einer Ausbeute von 80% und einer Reinheit von 98,9% erhalten werden. Die Identität und Reinheit des finalen Produktes wurde mittels NMR- und GC-MS-Analyse ermittelt (GC-Trennsäule: Agilent J&W DB-5, 30 m x 0,32 mm x 1,0 µm oder Ohio Valley OV-1701 60 m x 0,32 mm x 0,25 µm).

### Katalytische Hydrierung (= Schritt c1):

Eine 20 %-ige Lösung (Gew.-%) von Di-(n-butyl)-2,5-furandicarboxylat in THF wurde in einen stickstoffgefüllten 2,5 L Hastalloy-C Autoklaven der Firma Parr Instrument, bestückt mit einem mechanischen Rührer mit Magnetkupplung, Thermokupplung, Probeentnahmerohr und Stromstörer, gegeben. Anschließend wurden 120 g eines heterogenen Pd/Pt-Katalysators (0,4 Gew.-% Pd / 0,4 Gew.-% Pt auf ZrO₂, hergestellt analog zu DE4429014, Beispiel 6) zugegeben und die Stickstoffatmosphäre dreimal mit einem Überdruck Wasserstoff ausgetauscht. Der finale Wasserstoffdruck wurde auf 200 bar erhöht und der Autoklav auf 180°C erhitzt. Der Reaktionsverlauf wurde mittels GC-Analyse verfolgt. Nach vollständigem Umsatz (üblicherweise nach 40 bis 60 Stunden) wurde der Autoklav belüftet und der Inhalt abfiltriert, um den festen Katalysator zu entfernen. Das im Filtrat befindliche Lösungsmittel wurde anschließend unter reduziertem Druck destillativ entfernt, das zurückgebliebene Rohprodukt in 300 mL TBME verdünnt und in einen Scheidetrichter überführt. Die organische Phase wurde zweimal mit gesättigter NaHCO₃-Lösung und einmal mit gesättigter Kochsalzlösung gewaschen. Anschließend wurde das Lösungsmittel und andere leichtflüchtige Bestandteile unter reduziertem Druck destillativ entfernt. Das Rohprodukt wurde mittels fraktionierter Destillation gereinigt, worauf Di-(n-butyl)-2,5-tetrahydrofurandicarboxylat als farblose bis leicht braune, viskose Flüssigkeit in einer Ausbeute von 30% und einer Reinheit von 97,9% erhalten wurde. Die Identität und Reinheit des finalen Produktes wurde mittels NMR- und GC-MS-Analyse ermittelt (GC-Trennsäule: Agilent J&W DB-5, 30 m x 0,32 mm x 1,0 µm).

### Beispiel 3

### Synthese des Di-n-butylethers von 2,5-Di(hydroxymethyl)tetrahydrofuran

In einem 500 mL Vierhalskolben, ausgestattet mit einem mechanischen Rührer, Tropftrichter, Thermometer und Rückflusskühler, wurden 10,6 g 2,5-Di(hydroxymethyl)-tetrahydrofuran (80 mmol, 1,0 Äquivalent) in 140 ml Toluol gelöst. Dazu wurde bei Raumtemperatur über einen Zeitraum von 30 Minuten und unter ständigem Rühren portionenweise 22,4 g (400 mmol, 5,0 Äquivalente) gepudertes KOH zugegeben. Die Mischung wurde anschließend für 3 bis 4 Stunden unter Rückfluss gerührt. Dann wurden 60,0 g Molekularsieb (3Ä) zugegeben und für eine weitere Stunde unter Rückfluss gerührt, worauf eine cremefarbige Suspension erhalten wurde. Die Mischung wurde auf 90°C abgekühlt und 28,5 g (208 mmol, 2,6 Äquivalente) 1-Brombutan gelöst in 40 mL Toluol tropfenweise über 1,5 Stunden zugegeben. Der Tropftrichter wurde mit 20 mL Toluol gewaschen und die Waschlösung mit der Reaktionsmischung vereint. Der Reaktionsverlauf wurde mittels GC-Analyse verfolgt. Nach Beendigung der Reaktion (üblicherweise zwischen 40 und 80 Stunden) wurde die Mischung auf Raumtemperatur gekühlt. Die Glasbehältnisse wurden mit TBME gewaschen, mit der Reaktionsmischung vereint und die resultierende weiße Suspension filtriert. Die abfiltrierten Salzrückstände wurden mit TBME gewaschen. Die vereinigten organischen Phasen wurden nacheinander jeweils einmal mit gesättigter Kochsalzlösung, gesättigter Ammoniumchlorid-Lösung und wieder mit gesättigter Kochsalzlösung gewaschen und schließlich über Na₂SO₄ getrocknet. Anschließend wurde das Lösungsmittel und andere leichtflüchtige Bestandteile unter reduziertem Druck destillativ entfernt und der Rückstand am Hochvakuum getrocknet. Das Rohprodukt wurde mittels fraktionierter Destillation gereinigt, worauf der Di-n-butylether von 2,5-Di(hydroxymethyl)tetrahydrofuran als klare farblose Flüssigkeit in einer Ausbeute von 55% und einer Reinheit von 98,7% erhalten wurde. Die Identität und Reinheit des finalen Produkts wurde mittels NMR- und GC-MS-Analyse ermittelt (GC-Trennsäule: Agilent J&W DB-5, 30 m x 0,32 mm x 1,0 µm).

### II) Herstellung von weichgemachten PVC-Walzfolien und PVC-Probekörpern:

### II.a) Herstellung von PVC-Walzfolien:

Zur Beurteilung der weichmachenden Eigenschaften der erfindungsgemäßen Weichmacher und der Vergleichsverbindungen bei der thermoplastischen Verarbeitung wurden 0,5 mm dicke Weich-PVC-Folien hergestellt. Die Herstellung dieser Folien erfolgte durch Walzen und Pressen von weichgemachtem PVC.

Um Einflüsse durch unterschiedliche Additive zu vermeiden, wurde zur Herstellung des weichgemachten PVCs jeweils die nachfolgende Rezeptur verwendet:

| Additiv | phr |
|---|---|
| Solvin 271 SP¹⁾ | 100 |
| Weichmacher | 50 bzw. 70 |
| Reagens SLX 781²⁾ | 2 |

| | |
|---|---|
| 1) kommerziell erhältliches PVC der Firma Solvin GmbH & Co. KG, hergestellt durch Suspensionspolymerisation (K-Wert nach ISO 1628-2: 71) 2) flüssiger Ba-Zn-Stabilisator der Firma Reagens Deutschland GmbH | |

Die Zutaten wurden mit einem Handmixer bei Raumtemperatur vermischt. Die Mischung wurde anschließend auf einem dampfbeheizten Labormischwerk der Firma Collin (Typ "150") plastifiziert und zu einem Walzfell verarbeitet. Die Drehzahlen lagen bei 15 Umdrehungen/Minute (vordere Walze) und 12 Umdrehungen/Minute (hintere Walze) bei einer Walzzeit von 5 Minuten. Man erhielt so ein Walzfell mit einer Dicke von 0,55 mm. Das abgekühlte Walzfell wurde anschließend unter einem Druck von 220 bar innerhalb von 400 Sekunden auf einer Presse vom Typ "400P" der Firma Collin zu einer Weich-PVC-Folie mit der Dicke von 0,50 mm gepresst.

Die jeweiligen Walz- und Pressbedingungen können der nachfolgenden Tabelle entnommen werden:

| Bsp.-Nr. | Produkt | Weichmachergehalt [phr] | Walzen [°C] | Pressen [°C] |
|---|---|---|---|---|
| 1 | 2,5-THFDCS-di(n-butylester) | 50/70 | 160/160 | 160/160 |
| V1 | Mesamoll® TP-LXS 51067³⁾ | 50/70 | 175/165 | 185/175 |

| | | | | |
|---|---|---|---|---|
| 3) Mischung von Alkylsulfonsäurephenylestern der Lanxess Deutschland GmbH (CAS Nr. 91082-17-6) | | | | |

Aus den so hergestellten Walz- bzw. Pressfolien wurden die für die Prüfungen benötigten Probekörper hergestellt.

### II.b) Herstellung von Probekörpern:

Die Probekörper mit den Maßen 49 mm x 49 mm x 10 mm (Länge x Breite x Dicke) wurden aus Walzfolien durch Verpressen hergestellt, bei einer Temperatur die 10°C über der Walztemperatur lag. Für die anwendungstechnischen Prüfungen wurden die Probekörper 7 Tage bei 23°C und 50% relative Luftfeuchtigkeit gelagert.

### III) Anwendungstechnische Prüfungen:

### III.a) Bestimmung der Lösetemperatur nach DIN 53408:

Zur Charakterisierung des Gelierverhaltens der erfindungsgemäßen Weichmacher in PVC wurde die Lösetemperatur nach DIN 53408 bestimmt. Nach DIN 53408 wird ein Tropfen einer Aufschlämmung von 1 g PVC in 19 g Weichmacher unter einem mit einem heizbaren Mikroskoptisch ausgestatteten Mikroskop im durchscheinenden Licht beobachtet. Die Temperatur wird dabei ab 60°C linear um 2°C pro Minute erhöht. Als Lösetemperatur gilt die Temperatur, bei welcher die PVC-Teilchen unsichtbar werden, d. h. deren Konturen und Kontraste nicht mehr zu erkennen sind. Je niedriger die Lösetemperatur, desto besser ist das Gelierverhalten der betreffenden Substanz für PVC.

In der nachfolgenden Tabelle sind die Lösetemperaturen des erfindungsgemäßen Weichmachers Di(n-butyl)-2,5-tetrahydrofurandicarboxylat und als Vergleich von Mesamoll® TP-LXS 5106 sowie von Dibutylphthalat aufgeführt.

| Bsp.-Nr. | Substanz | Lösetemperatur nach DIN 53408 |
|---|---|---|
| | | [°C] |
| 1 | Di(n-butyl)-2,5-tetrahydrofurandicarboxylat | 71 |
| V1 | Mesamoll® TP-LXS 51067³⁾ | 114 |
| V2 | Dibutylphthalat⁴⁾ | 100 |

| | | |
|---|---|---|
| 3) Mischung von Alkylsulfonsäurephenylestern der Lanxess Deutschland GmbH (CAS Nr. 91082-17-6) 4) Benzol-1,2-dicarbonsäure-di(n-butyl)ester (CAS Nr. 84-74-2) | | |

Wie aus der Tabelle ersichtlich, zeigt der erfindungsgemäße Weichmacher die niedrigste Lösetemperatur.

### III.b) Physikalische Kenndaten:

In der nachfolgenden Tabelle sind die wesentlichsten physikalischen Kenndaten von Di(n-butyl)-2,5-tetrahydrofurandicarboxylat (Beispiel 1) im Vergleich zu dem am Markt verwendeten Weichmachers Mesamoll® TP-LXS 51067 (Vergleichsbeispiel V1) aufgeführt.

| Weichmacher: | Di(n-butyl)-2,5-tetrahydrofurandicarboxylat | Mesamoll® TP-LXS 51067 |
|---|---|---|
| Dichte (20°C) [g/cm³] | 1,048 | 1,071 |
| Viskosität (20°C) [mPas] | 10 | 90 |

Von den physikalischen Eigenschaften sind neben der Lösetemperatur nach DIN 53408 speziell die Dichte und Viskosität für die Anwendung als Weichmacher relevant. Im Vergleich mit dem kommerziell erhältlichen und hinsichtlich seinen Eigenschaften als günstig bewerteten Weichmacher Mesamoll® TP-LXS 51067 zeigt der 2,5-THFDCS-Dibutylester eine deutlich niedrigere und damit günstigere Viskosität bei vergleichbarer Dichte.

### III.c) Bestimmung der Shore-Härte:

Die Bestimmung der Shore-Härte A bzw. D erfolgte nach DIN EN ISO 868 mit einem Digital-Durometer Modell DD-3 der Firma Hildebrand. Die Probekörper wurden gemäß Beispiel II.c) hergestellt. Die in der Figur 1 und Figur 2 gezeigten Werte stellen jeweils den Mittelwert aus 20 Messungen pro Probekörper (je 10 Messungen auf der Vorder- und der Rückseite) dar. Der Messwert wurde immer nach einer Zeit von 15 Sekunden ermittelt.

Wie aus den Diagrammen der Figur 1 und Figur 2 ersichtlich, zeigt der erfindungsgemäße 2,5-THFDCS-dibutylester eine deutlich bessere weichmachende Wirkung als der kommerziell erhältliche Weichmacher Mesamoll® TP-LXS 51067.

### III.d) Bestimmung des 100%-Moduls:

Das 100%-Modul ist neben der Shore-Härte eine weitere Eigenschaft, die die weichmachende Wirkung von Weichmachern, d.h. die Weichmacher-Effizienz, charakterisiert.

Die Bestimmung des 100%-Moduls erfolgte nach DIN EN ISO 527 Teil 1 und 3 mit einer Prüfvorrichtung der Fa. Zwick Typ TMZ 2.5/TH1S. Die Probekörper mit den Maßen 150mm x 10mm x 0,5mm (Länge x Breite x Dicke) entsprechen dem Typ 2 nach DIN EN ISO 527 Teil 3 und werden aus den Walz/Pressfolien mittels eines Stanzeisens ausgestanzt. Vor der Prüfung werden die Probekörper 7 Tage konditioniert. Die Konditionierung und die Zugprüfungen erfolgen bei 23 °C +/- 1,0 °C und 50% +/- 5 relativer Feuchte gemäß DIN EN ISO 291. Die in der Figur 3 aufgetragenen Werte stellen jeweils Mittelwerte aus der Prüfung von 10 einzelnen Probekörpern dar.

Wie aus dem Diagramm der Figur 3 ersichtlich, zeigt der erfindungsgemäße 2,5-THFDCS-dibutylester eine deutlich bessere weichmachende Wirkung als der kommerziell erhältliche Weichmacher Mesamoll® TP-LXS 51067.

### III.e) Bestimmung der Kälteflexibilität:

Zur Bestimmung der Kälteflexibilität wurden PVC-Folien verwendet, die den jeweiligen zu testenden Weichmacher in unterschiedlichen Konzentrationen enthielt. Es wurden 2 Methoden eingesetzt. Zum einen erfolgte die Bestimmung der Kältebruchtemperatur in Anlehnung an die nicht mehr aktuelle Norm DIN 53372, zum anderen wurde die Glasübergangstemperatur T_{g} der Folien mittels DMA (dynamisch mechanische Analyse) nach ISO 6721-7 aus dem Maximum des Verlustmoduls "G" ermittelt. Die Ergebnisse der beiden Testmethoden sind in den Figuren 4 und 5 dargestellt.

Wie aus den Diagrammen der Figuren 4 und Figur 5 hervorgeht, zeigen die PVC-Folien die den erfindungsgemäßen 2,5-THFDCS-dibutylester enthalten im Vergleich zu PVC-Folien mit Mesamoll® TP-LXS 51067 eine niedrigere und somit günstigere Kältebruchtemperatur. Dasselbe gilt für die Glasübergangstemperatur. Überraschend ist der exzellente Wert für die Glasübergangstemperatur des erfindungsgemäßen 2,5-THFDCS-dibutylesters bei 70 phr Weichmachergehalt.

## Patentansprüche

1. Verwendung wenigstens einer Verbindung der allgemeinen Formel (I) 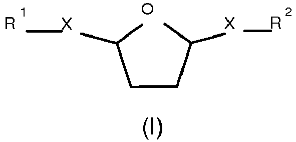 worin
X für *-(C=O)-O-, *-(CH₂)ₙ-O- oder *-(CH₂)ₙ-O-(C=O)- steht, wobei * den Verknüpfungspunkt mit dem Tetrahydrofuranring darstellt und n den Wert 0, 1 oder 2 aufweist;
und
R¹ und R² unabhängig voneinander ausgewählt sind unter C₄-C₅-Alkyl und C₅-C₆-Cycloalkyl, wobei die Cycloalkylreste unsubstituiert sind oder durch wenigstens einen C₁-C₁₀-Alkylrest substituiert sein können,
oder einer Weichmacher-Zusammensetzung, enthaltend wenigstens eine Verbindung der allgemeinen Formel (I), wie oben definiert, und wenigstens einen von den Verbindungen der Formel (I) verschiedenen Weichmacher, als Weichmacher für thermoplastische Polymere und Elastomere.

2. Verwendung nach Anspruch 1, wobei in den Verbindungen der allgemeinen Formel (I) R¹ und R² unabhängig voneinander für einen unverzweigten oder verzweigten C₄-Alkylrest stehen.

3. Verwendung nach einem der vorangehenden Ansprüche, wobei in den Verbindungen der allgemeinen Formel (I) die Gruppe X beide für *-(C=O)-O- steht.

4. Verwendung wenigstens einer Verbindung der allgemeinen Formel (I), wie in einem der Ansprüche 1 bis 3 definiert, oder einer Weichmacher-Zusammensetzung, enthaltend wenigstens eine Verbindung der allgemeinen Formel (I), wie in einem der Ansprüche 1 bis 3 definiert, und wenigstens einen von den Verbindungen der Formel (I) verschiedenen Weichmacher, als Weichmacher für ein thermoplastisches Polymer, das Polyvinylchlorid enthält oder aus Polyvinylchlorid besteht.

5. Verwendung wenigstens einer Verbindung der allgemeinen Formel (I), wie in einem der Ansprüche 1 bis 3 definiert, oder einer Weichmacher-Zusammensetzung, enthaltend wenigstens eine Verbindung der allgemeinen Formel (I), wie in einem der Ansprüche 1 bis 3 definiert, und wenigstens einen von den Verbindungen der Formel (I) verschiedenen Weichmacher, als Weichmacher für ein Elastomer, das ein natürlichen und/oder synthetischen Kautschuk enthält oder aus einem natürlichen und/oder synthetischen Kautschuk besteht.

6. Verwendung wenigstens einer Verbindung der allgemeinen Formel (I), wie in einem der Ansprüchen 1 bis 3 definiert, oder einer Weichmacher-Zusammensetzung, enthaltend wenigstens eine Verbindung der allgemeinen Formel (I), wie in einem der Ansprüche 1 bis 3 definiert, und wenigstens einen von den Verbindungen der Formel (I) verschiedenen Weichmacher, als Weichmacher in einem Plastisol.

7. Verwendung wenigstens einer Verbindung der allgemeinen Formel (I), wie in einem der Ansprüche 1 bis 3 definiert, als Weichmacher zur Verringerung der zum Gelieren eines thermoplastischen Polymers erforderlichen Temperatur und/oder zur Erhöhung der Geliergeschwindigkeit.

8. Verwendung nach einem der Ansprüche 1 bis 7, wobei der von den Verbindungen (I) verschiedene Weichmacher ausgewählt ist unter Phthalsäuredialkylestern, Phthalsäurealkylaralkylestern, Terephthalsäuredialkylestern, Trimellithsäuretrialkylestern, Adipinsäuredialkylestern, Benzoesäurealkylestern, Dibenzoesäureestern von Glycolen, Hydroxybenzoesäureestern, Estern von gesättigten Mono- und Dicarbonsäuren, Estern von ungesättigten Dicarbonsäuren, Amiden und Estern von aromatischen Sulfonsäuren, Alkylsulfonsäureestern, Glycerinestern, Isosorbidestern, Phosphorsäureestern, Citronensäuretriestern, Alkylpyrrolidonderivaten, 2,5-Furandicarbonsäureestern, epoxidierten Pflanzenölen auf Basis von Triglyceriden und gesättigten oder ungesättigten Fettsäuren, Polyestern aus aliphatischen und/oder aromatischen Polycarbonsäuren mit wenigstens zweiwertigen Alkoholen.

9. Weichmacher-Zusammensetzung, enthaltend wenigstens eine Verbindung der allgemeinen Formel (I), wie in einem der Ansprüche 1 bis 3 definiert, und wenigstens einen von den Verbindungen der Formel (I) verschiedenen Weichmacher.

10. Weichmacher-Zusammensetzung nach Anspruch 9, wobei der von den Verbindungen (I) verschiedene Weichmacher ausgewählt ist unter Weichmachern wie in Anspruch 8 definiert.

11. Verbindungen der allgemeinen Formel (I) 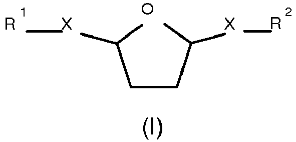 worin
X für *-(C=O)-O-, *-(CH₂)₂-O- oder *-(CH₂)-O-(C=O)- steht, wobei * den Verknüpfungspunkt mit dem Tetrahydrofuranring darstellt;
und
R¹ und R² unabhängig voneinander ausgewählt sind unter n-Butyl oder Isobutyl.

12. Verbindungen nach Anspruch 11, wobei die Gruppe X beide für *-(C=O)-O-steht.

13. Formmasse enthaltend wenigstens ein Polymer und wenigstens eine Verbindung der allgemeinen Formel (I), wie in einem der Ansprüche 11 oder 12 definiert.

14. Formmasse enthaltend wenigstens ein Polymer und eine Weichmacher-Zusammensetzung, wie in einem der Ansprüche 9 oder 10 definiert.

15. Formmasse nach Anspruch 13 oder 14, wobei es sich bei dem Polymer um ein thermoplastisches Polymer handelt, das ausgewählt ist unter
- Homo- und Copolymeren, die wenigstens ein Monomer einpolymerisiert enthalten, das ausgewählt ist unter C₂-C₁₀-Monoolefinen, 1,3-Butadien, 2-Chlor-1,3-butadien, Vinylalkohol und dessen C₂-C₁₀-Alkylestern, Vinylchlorid, Vinylidenchlorid, Vinylidenfluorid, Tetrafluorethylen, Glycidylacrylat, Glycidylmethacrylat, Acrylaten und Methacrylaten von C₁-C₁₀-Alkoholen, Vinylaromaten, (Meth)acrylnitril, Maleinsäureanhydrid und α,β-ethylenisch ungesättigten Mono- und Dicarbonsäuren,
- Homo- und Copolymeren von Vinylacetalen,
- Polyvinylestern,
- Polycarbonaten,
- Polyestern,
- Polyethern,
- Polyetherketonen,
- thermoplastischen Polyurethanen,
- Polysulfiden,
- Polysulfonen,
- Polyethersulfonen,
- Cellulosealkylestern,
und Mischungen davon.

16. Formmasse nach Anspruch 15, wobei es sich bei dem thermoplastischen Polymer um Polyvinylchlorid (PVC) handelt.

17. Formmasse nach Anspruch 16, enthaltend wenigstens eine Verbindung der allgemeinen Formel (I), wie in einem der Ansprüche 11 oder 12 definiert, oder eine Weichmacher-Zusammensetzung, wie in einem der Ansprüche 9 oder 10 definiert, wobei der Gesamtweichmachergehalt 1,0 bis 400 phr beträgt.

18. Formmasse nach Anspruch 15, enthaltend wenigstens ein von Polyvinylchlorid verschiedenes thermoplastisches Polymer, wenigstens eine Verbindung der allgemeinen Formel (I), wie in einem der Ansprüche 11 oder 12 definiert, oder eine Weichmacher-Zusammensetzung, wie in einem der Ansprüche 9 oder 10 definiert, wobei der Gesamtweichmachergehalt 0,5 bis 300 phr beträgt.

19. Verfahren zur Herstellung von Verbindungen der allgemeinen Formel (I.1), 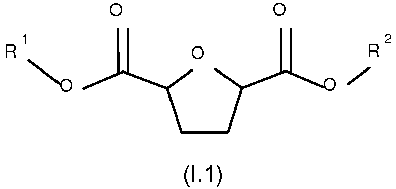 worin
R¹ und R² unabhängig voneinander ausgewählt sind unter n-Butyl oder Isobutyl, bei dem man
a) gegebenenfalls 2,5-Furandicarbonsäure oder ein Anhydrid oder Säurehalogenid davon mit einem C₁-C₃-Alkanol in Gegenwart eines Katalysators unter Erhalt eines Di-(C₁-C₃-Alkyl)-2,5-furandicarboxylats umsetzt,
b1) 2,5-Furandicarbonsäure oder ein Anhydrid oder Säurehalogenid davon oder das in Schritt a) erhaltene Di-(C₁-C₃-Alkyl)-2,5-furandicarboxylat mit n-Butanol und/oder Isobutanol in Gegenwart wenigstens eines Katalysators unter Erhalt einer Verbindung der Formel (I.1a) umsetzt, 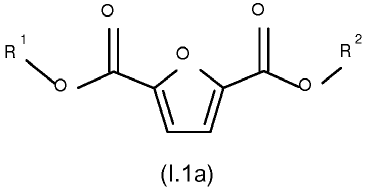
c1) die in Schritt b1) erhaltene Verbindung (I.1a) mit Wasserstoff in Gegenwart wenigstens eines Hydrierkatalysators unter Erhalt der Verbindung der allgemeinen Formel (I.1) hydriert,
oder
b2) 2,5-Furandicarbonsäure oder das in Schritt a) erhaltene 2,5-Di-(C₁-C₃-Alkyl)furandicarboxylat mit Wasserstoff in Gegenwart wenigstens eines Hydrierkatalysators unter Erhalt einer Verbindung der allgemeinen Formel (1.1b) hydriert, 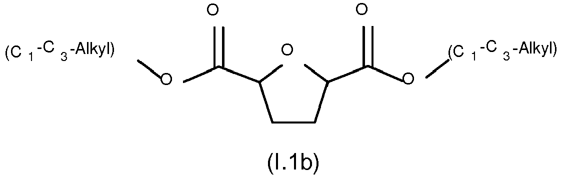
c2) die in Schritt b2) erhaltene Verbindung (I.1b) mit n-Butanol und/oder Isobutanol in Gegenwart eines Katalysators unter Erhalt einer Verbindung der Formel (I.1) umsetzt.

20. Verfahren zur Herstellung von Verbindungen der allgemeinen Formel (I.2) oder (I.3), 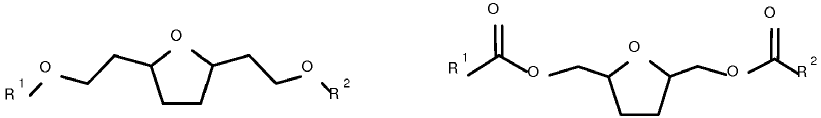 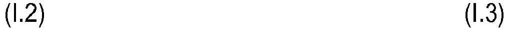 worin
R¹ und R² unabhängig voneinander ausgewählt sind unter n-Butyl oder Isobutyl, bei dem man
a) 2,5-Di-(hydroxyethyl)tetrahydrofuran mit wenigstens einem Alkylierungsreagenz R¹-Z und, falls R¹ und R² unterschiedliche Bedeutung haben, zusätzlich mit wenigstens einem Alkylierungsreagenz R²-Z, wobei Z für eine Abgangsgruppe steht, in Gegenwart einer Base in Verbindungen der Formel (I.2) umsetzt,
oder
b) 2,5-Di-(hydroxymethyl)tetrahydrofuran mit wenigstens einem Säurehalogenid R¹-(C=O)X und, falls R¹ und R² unterschiedliche Bedeutung haben, mit wenigstens einem Säurehalogenid R²-(C=O)X, wobei X für Br oder Cl steht, in Gegenwart wenigstens eines tertiären Amins in Verbindungen der Formel (I.3) umsetzt.

21. Verfahren nach Anspruch 20, wobei die Abgangsgruppe Z für eine Rest steht, der ausgewählt ist unter Br, Cl, der Tosyl-, Mesyl- oder Triflyl-Gruppe.

22. Verwendung einer Formmasse, wie in einem der Ansprüche 13 bis 18 definiert, zur Herstellung von Formkörpern und Folien, wie beispielsweise Gehäusen von Elektrogeräten, Computergehäusen, Werkzeugen, Rohrleitungen, Kabeln, Schläuchen, Draht-Ummantelungen, Fensterprofilen, Komponenten für den Fahrzeugbau, Reifen, Möbeln, Schaumstoff für Polster und Matratzen, Planen, Dichtungen, Verbundfolien, Schallplatten, Kunstleder, Verpackungsbehältern, Klebebandfolien oder Beschichtungen.

23. Verwendung einer Formmasse wie in einem der Ansprüche 13 bis 18 definiert zur Herstellung von Formkörpern und Folien, die direkt mit Menschen oder Nahrungsmitteln in Kontakt kommen.

## Claims

1. The use of at least one compound of the general formula (I) 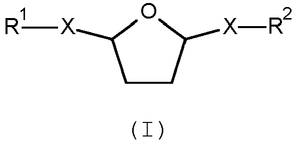 in which
X is *-(C=O)-O-, *- (CH₂)ₙ-O- or *-(CH₂)ₙ-O-(C=O)-, where * is the point of linkage to the tetrahydrofuran ring, and n has the value 0, 1, or 2;
and
R¹ and R² have been selected mutually independently from C₄-C₅-alkyl and C₅-C₆-cycloalkyl, where the cycloalkyl moieties are unsubstituted or can have substitution by at least one C₁-C₁₀-alkyl moiety,
or of a plasticizer composition comprising at least one compound of the general formula (I) as defined above, and at least one plasticizer different from the compounds of the formula (I), as plasticizer for thermoplastic polymers and elastomers.

2. The use according to claim 1, where, in the compounds of the general formula (I), R¹ and R² are mutually independently an unbranched or branched C₄-alkyl moiety.

3. The use according to either of the preceding claims, where, in the compounds of the general formula (I), both of the groups X are *-(C=O)-O-.

4. The use of at least one compound of the general formula (I) as defined in any of claims 1 to 3, or of a plasticizer composition comprising at least one compound of the general formula (I) as defined in any of claims 1 to 3 and at least one plasticizer different from the compounds of the formula (I), as plasticizer for a thermoplastic polymer which comprises polyvinyl chloride or is composed of polyvinyl chloride.

5. The use of at least one compound of the general formula (I) as defined in any of claims 1 to 3, or of a plasticizer composition comprising at least one compound of the general formula (I) as defined in any of claims 1 to 3 and at least one plasticizer different from the compounds of the formula (I), as plasticizer for an elastomer which comprises a natural and/or synthetic rubber or is composed of a natural and/or synthetic rubber.

6. The use of at least one compound of the general formula (I) as defined in any of claims 1 to 3, or of a plasticizer composition comprising at least one compound of the general formula (I) as defined in any of claims 1 to 3 and at least one plasticizer different from the compounds of the formula (I), as plasticizer in a plastisol.

7. The use of at least one compound of the general formula (I) as defined in any of claims 1 to 3, as plasticizer for reducing the temperature required for the gelling of a thermoplastic polymer, and/or for increasing the gelling rate.

8. The use according to any of claims 1 to 7, where the plasticizer different from the compounds (I) has been selected from dialkyl phthalates, alkyl aralkyl phthalates, dialkyl terephthalates, trialkyl trimellitates, dialkyl adipates, alkyl benzoates, dibenzoic esters of glycols, hydroxybenzoic esters, esters of saturated mono- and dicarboxylic acids, esters of unsaturated dicarboxylic acids, amides and esters of aromatic sulfonic acids, alkylsulfonic esters, glycerol esters, isosorbide esters, phosphoric esters, citric triesters, alkylpyrrolidone derivatives, 2,5-furandicarboxylic esters, epoxidized vegetable oils based on triglycerides and saturated or unsaturated fatty acids, polyesters derived from aliphatic and/or aromatic polycarboxylic acids with at least dihydric alcohols.

9. A plasticizer composition comprising at least one compound of the general formula (I) as defined in any of claims 1 to 3 and at least one plasticizer different from the compounds of the formula (I).

10. The plasticizer composition according to claim 9, where the plasticizer different from the compounds (I) has been selected from plasticizers as defined in claim 8.

11. A compound of the general formula (I) 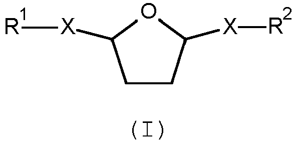 in which
X is *-(C=O)-O-, *- (CH₂)₂-O- or *-(CH₂)-O-(C=O)-, where * is the point of linkage to the tetrahy-drofuran ring;
and
R¹ and R² have been selected mutually independently from n-butyl or isobutyl.

12. The compound according to claim 11, where both the groups X are *-(C=O)-O-.

13. A molding composition comprising at least one polymer and at least one compound of the general formula (I) as defined in either of claims 11 and 12.

14. A molding composition comprising at least one polymer and a plasticizer composition as defined in either of claims 9 and 10.

15. The molding composition according to claim 13 or 14, where the polymer involved is a thermoplastic polymer selected from
- homo- and copolymers which comprise at least one copolymerized monomer selected from C₂-C₁₀-monoolefins, 1,3-butadiene, 2-chloro-1,3-butadiene, vinyl alcohol and its C₂-C₁₀-alkyl esters, vinyl chloride, vinylidene chloride, vinylidene fluoride, tetrafluoroethylene, glycidyl acrylate, glycidyl methacrylate, acrylates and methacrylates of C₁-C₁₀- alcohols, vinylaromatics, (meth)acrylonitrile, maleic anhydride, and α,β-ethylenically unsaturated mono- and dicarboxylic acids,
- homo- and copolymers of vinyl acetals,
- polyvinyl esters,
- polycarbonates,
- polyesters,
- polyethers,
- polyether ketones,
- thermoplastic polyurethanes,
- polysulfides,
- polysulfones,
- polyether sulfones,
- cellulose alkyl esters,
and mixtures thereof.

16. The molding composition according to claim 15, where the thermoplastic polymer involved is polyvinyl chloride (PVC).

17. The molding composition according to claim 16, comprising at least one compound of the general formula (I) as defined in either of claims 11 and 12 or a plasticizer composition as defined in either of claims 9 and 10, where the total plasticizer content is from 1.0 to 400 phr.

18. The molding composition according to claim 15, comprising at least one thermoplastic polymer different from polyvinyl chloride, at least one compound of the general formula (I) as defined in either of claims 11 and 12 or a plasticizer composition as defined in either of claims 9 and 10, where the total plasticizer content is from 0.5 to 300 phr.

19. A process for producing compounds of the general formula (I.1), 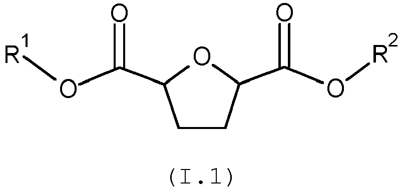 in which
R¹ and R² have been selected mutually independently from n-butyl or isobutyl,
where
a) optionally 2,5-furandicarboxylic acid or an anhydride or acyl halide thereof is reacted with a C₁-C₃-alkanol in the presence of a catalyst to give a di (C₁-C₃-alkyl) 2,5-furandicarboxylate,
b1) 2,5-furandicarboxylic acid or an anhydride or acyl halide thereof, or the di(C₁-C₃-alkyl) 2,5-furandicarboxylate obtained in step a), is reacted with n-butanol and/or isobutanol in the presence of at least one catalyst to give a compound of the formula (I.1a), 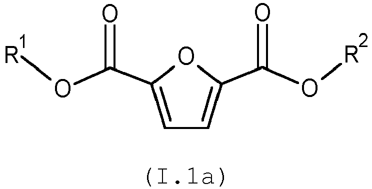
c1) the compound (I.1a) obtained in step b1) is hydrogenated with hydrogen in the presence of at least one hydrogenation catalyst to give the compound of the general formula (1.1),
or
b2) 2,5-furandicarboxylic acid or the di(C₁-C₃-alkyl) 2,5-furandicarboxylate obtained in step a) is hydrogenated with hydrogen in the presence of at least one hydrogenation catalyst to give a compound of the general formula (I.1b), 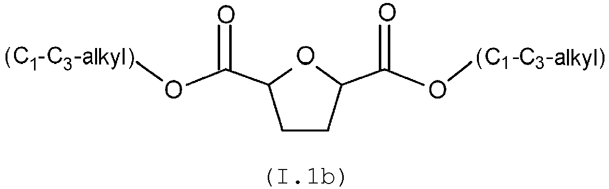
c2) the compound (I.1b) obtained in step b2) is reacted with n-butanol and/or isobutanol in the presence of a catalyst to give a compound of the formula (1.1).

20. A process for producing compounds of the general formula (1.2) or (1.3), 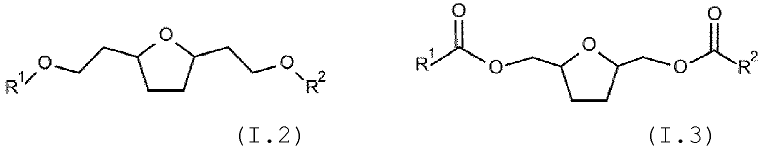 in which
R¹ and R² have been selected mutually independently from n-butyl or isobutyl,
where
a) 2,5-di(hydroxyethyl)tetrahydrofuran is reacted with at least one alkylating reagent R¹-Z and, if R¹ and R² are different, also with at least one alkylating reagent R²-Z, where Z is a leaving group, in the presence of a base to give compounds of the formula (1.2),
or
b) 2,5-di(hydroxymethyl)tetrahydrofuran is reacted with at least one acyl halide R¹-(C=O)X and, if R¹ and R² are different, with at least one acyl halide R²-(C=O)X, where X is Br or Cl, in the presence of at least one tertiary amine to give compounds of the formula (1.3).

21. The process according to claim 20, where the leaving group Z is a moiety selected from Br, Cl, and the tosyl, mesyl or triflyl group.

22. The use of a molding composition as defined in any of claims 13 to 18 for producing moldings and foils, for example housings of electrical devices, computer housings, tooling, piping, cables, hoses, wire sheathing, window profiles, vehicle-construction components, tires, furniture, cushion foam and mattress foam, tarpaulins, gaskets, composite foils, recording disks, synthetic leather, packaging containers, adhesive-tape foils, or coatings.

23. The use of a molding composition as defined in any of claims 13 to 18 for producing moldings and foils which come directly into contact with people or with foods.

## Revendications

1. Utilisation d'au moins un composé de formule générale (I) 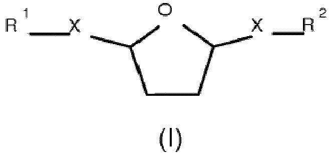 dans laquelle
X représente *-(C=O)-O-, *-(CH₂)ₙ-O- ou *-(CH₂)ₙ-O-(C=O)-, * représentant le point de liaison avec le cycle tétrahydrofurane et n présente la valeur 0, 1 ou 2 ;
et
R¹ et R² sont choisis indépendamment l'un de l'autre parmi alkyle en C₄-C₅ et cycloalkyle en C₅-C₆, les radicaux cycloalkyle étant non substitués ou pouvant être substitués par au moins un radical alkyle en C₁-C₁₀,
ou d'une composition de plastifiant, contenant au moins un composé de formule générale (I), tel que défini précédemment, et au moins un plastifiant différent des composés de formule (I), en tant que plastifiant pour des polymères thermoplastiques et des élastomères.

2. Utilisation selon la revendication 1, dans laquelle, dans les composés de formule générale (I), R¹ et R² représentent indépendamment l'un de l'autre un radical alkyle en C₄ non ramifié ou ramifié.

3. Utilisation selon l'une quelconque des revendications précédentes, dans laquelle, dans les composés de formule générale (I), les groupes X représentent tous les deux *-(C=O)-O-.

4. Utilisation d'au moins un composé de formule générale (I), tel que défini dans l'une quelconque des revendications 1 à 3, ou d'une composition de plastifiant, contenant au moins un composé de formule générale (I), tel que défini dans l'une quelconque des revendications 1 à 3, et au moins un plastifiant différent des composés de formule (I), en tant que plastifiant pour un polymère thermoplastique, qui contient du polychlorure de vinyle ou est constitué de polychlorure de vinyle.

5. Utilisation d'au moins un composé de formule générale (I), tel que défini dans l'une quelconque des revendications 1 à 3, ou d'une composition de plastifiant, contenant au moins un composé de formule générale (I), tel que défini dans l'une quelconque des revendications 1 à 3, et au moins un plastifiant différent des composés de formule (I), en tant que plastifiant pour un élastomère qui contient un caoutchouc naturel et/ou synthétique, ou est constitué d'un caoutchouc naturel et/ou synthétique.

6. Utilisation d'au moins un composé de formule générale (I), tel que défini dans l'une quelconque des revendications 1 à 3, ou d'une composition de plastifiant, contenant au moins un composé de formule générale (I), tel que défini dans l'une quelconque des revendications 1 à 3, et au moins un plastifiant différent des composés de formule (I), en tant que plastifiant dans un plastisol.

7. Utilisation d'au moins un composé de formule générale (I), tel que défini dans l'une quelconque des revendications 1 à 3, en tant que plastifiant pour réduire la température nécessaire pour la gélification d'un polymère thermoplastique et/ou pour augmenter la vitesse de gélification.

8. Utilisation selon l'une quelconque des revendications 1 à 7, dans laquelle le plastifiant différent des composés (I) est choisi parmi les esters dialkyliques de l'acide phtalique, les esters alkylaralkyliques de l'acide phtalique, les esters dialkyliques de l'acide téréphtalique, les esters trialkyliques de l'acide triméllitique, les esters dialkyliques de l'acide adipique, les esters alkyliques de l'acide benzoïque, les esters de l'acide dibenzoïque de glycols, les esters de l'acide hydroxybenzoïque, les esters d'acides mono- et dicarboxyliques saturés, les esters d'acides dicarboxyliques insaturés, les amides et les esters d'acides sulfoniques aromatiques, les esters d'acides alkylsulfoniques, les esters de glycérine, les esters d'isosorbide, les esters d'acide phosphorique, les triesters d'acide citrique, les dérivés d'alkylpyrrolidone, les esters de l'acide 2,5-furane-dicarboxylique, les huiles végétales époxydées à base de triglycérides et d'acides gras saturés ou insaturés, les polyesters d'acides polycarboxyliques aliphatiques et/ou aromatiques avec des alcools au moins bivalents.

9. Composition de plastifiant, contenant au moins un composé de formule générale (I), tel que défini dans l'une quelconque des revendications 1 à 3, et au moins un plastifiant différent des composés de formule (I).

10. Composition de plastifiant selon la revendication 9, dans laquelle le plastifiant différent des composés (I) est choisi parmi les plastifiants tels que définis dans la revendication 8.

11. Composés de formule générale (I) 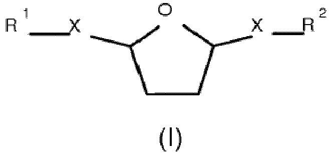 dans laquelle
X représente *-(C=O)-O-, *-(CH₂)₂-O- ou *-(CH₂)-O-(C=O)-, * représentant le point de liaison avec le cycle tétrahydrofurane ;
et
R¹ et R² sont choisis indépendamment l'un de l'autre parmi n-butyle ou isobutyle.

12. Composés selon la revendication 11, dans lesquels les groupes X représentent tous les deux *-(C=O)-O-.

13. Matériau de moulage contenant au moins un polymère et au moins un composé de formule générale (I), tel que défini dans l'une quelconque des revendications 11 ou 12.

14. Matériau de moulage contenant au moins un polymère et une composition de plastifiant, telle que définie dans l'une quelconque des revendications 9 ou 10.

15. Matériau de moulage selon la revendication 13 ou 14, dans lequel le polymère est un polymère thermoplastique, qui est choisi parmi
- les homo- et copolymères, qui contiennent au moins un monomère sous forme copolymérisée qui est choisi parmi les monooléfines en C₂-C₁₀, le 1,3-butadiène, le 2-chloro-1,3-butadiène, l'alcool vinylique et ses esters alkyliques en C₂-C₁₀, le chlorure de vinyle, le chlorure de vinylidène, le fluorure de vinylidène, le tétrafluoroéthylène, l'acrylate de glycidyle, le méthacrylate de glycidyle, les acrylates et les méthacrylates d'alcools en C₁-C₁₀, les composés aromatiques de vinyle, le (méth)acrylonitrile, l'anhydride de l'acide maléique et les acides mono- et dicarboxyliques α,β-éthyléniquement insaturés,
- les homo- et copolymères de vinylacétals,
- les polyesters de vinyle,
- les polycarbonates,
- les polyesters,
- les polyéthers,
- les polyéther-cétones,
- les polyuréthanes thermoplastiques,
- les polysulfures,
- les polysulfones,
- les polyéther-sulfones,
- les esters alkyliques de cellulose
et leurs mélanges.

16. Matériau de moulage selon la revendication 15, dans lequel le polymère thermoplastique est le polychlorure de vinyle (PVC).

17. Matériau de moulage selon la revendication 16, contenant au moins un composé de formule générale (I), tel que défini dans l'une quelconque des revendications 11 ou 12, ou une composition de plastifiant, telle que définie dans l'une quelconque des revendications 9 ou 10, la teneur totale en plastifiant étant de 1,0 à 400 pce.

18. Matériau de moulage selon la revendication 15, contenant au moins un polymère thermoplastique différent du polychlorure de vinyle, au moins un composé de formule générale (I), tel que défini dans l'une quelconque des revendications 11 ou 12, ou une composition de plastifiant, telle que définie dans l'une quelconque des revendications 9 ou 10, la teneur totale en plastifiant étant de 0,5 à 300 pce.

19. Procédé de fabrication de composés de formule générale (I.1) 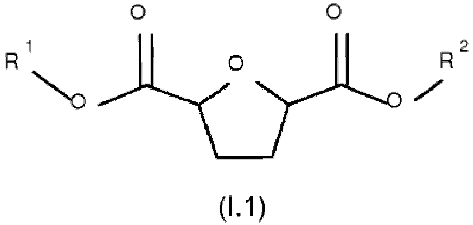 dans laquelle
R¹ et R² sont choisis indépendamment l'un de l'autre parmi n-butyle ou isobutyle,
selon lequel
a) de l'acide 2,5-furane-dicarboxylique ou un anhydride ou halogénure d'acide de celui-ci est éventuellement mis en réaction avec un alcanol en C₁-C₃ en présence d'un catalyseur pour obtenir un dicarboxylate de di-(alkyle en C₁-C₃)-2,5-furane,
b1) de l'acide 2,5-furane-dicarboxylique ou un anhydride ou halogénure d'acide de celui-ci ou le dicarboxylate de di-(alkyle en C₁-C₃)-2,5-furane obtenu à l'étape a) est mis en réaction avec du n-butanol et/ou de l'isobutanol en présence d'au moins un catalyseur pour obtenir un composé de formule (I.1a) 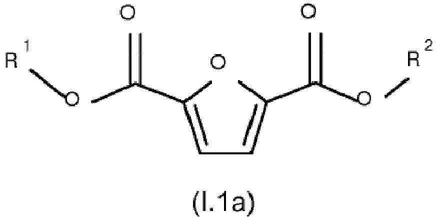
c1) le composé (I.1a) obtenu à l'étape b1) est hydrogéné avec de l'hydrogène en présence d'au moins un catalyseur d'hydrogénation pour obtenir le composé de formule générale (I.1),
ou
b2) de l'acide 2,5-furane-dicarboxylique ou le dicarboxylate de 2,5-di-(alkyle en C₁-C₃)-furane obtenu à l'étape a) est hydrogéné avec de l'hydrogène en présence d'au moins un catalyseur d'hydrogénation pour obtenir un composé de formule générale (I.1b), 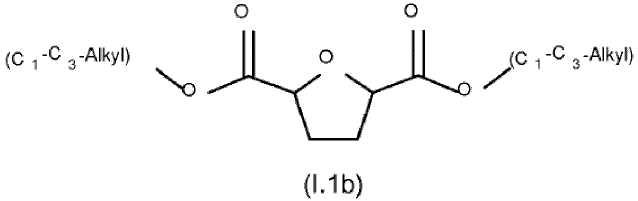
c2) le composé (I.1b) obtenu à l'étape b2) est mis en réaction avec du n-butanol et/ou de l'isobutanol en présence d'un catalyseur pour obtenir un composé de formule (I.1).

20. Procédé de fabrication de composés de formule générale (1.2) ou (1.3) 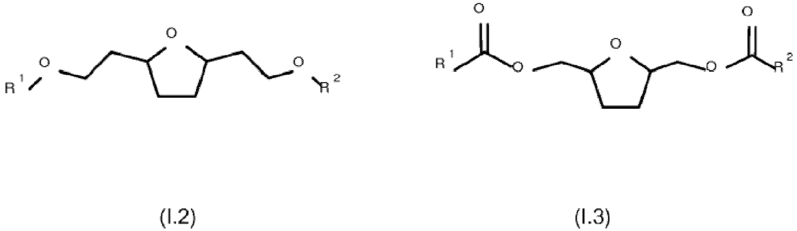 dans lesquelles
R¹ et R² sont choisis indépendamment l'un de l'autre parmi n-butyle ou isobutyle,
selon lequel
a) du 2,5-di(hydroxyéthyl)tétrahydrofurane est mis en réaction avec au moins un réactif d'alkylation R¹-Z et, si R¹ et R² ont une signification différente, en outre avec au moins un réactif d'alkylation R²-Z, Z représentant un groupe partant, en présence d'une base pour former des composés de formule (1.2)
ou
b) du 2,5-di(hydroxyméthyl)tétrahydrofurane est mis en réaction avec au moins un halogénure d'acide R¹-(C=O)X et, si R¹ et R² ont une signification différente, en outre avec au moins un halogénure d'acide R²-(C=O)X, X représentant Br ou Cl, en présence d'au moins une amine tertiaire pour former des composés de formule (I.3).

21. Procédé selon la revendication 20, dans lequel le groupe partant Z représente un radical qui est choisi parmi Br, Cl, le groupe tosyle, mésyle ou triflyle.

22. Utilisation d'un matériau de moulage, tel que défini dans l'une quelconque des revendications 13 à 18, pour la fabrication de corps moulés et de films, tels que par exemple des boîtiers d'appareils électriques, des boîtiers d'ordinateurs, des outils, des canalisations, des câbles, des tuyaux, des gainages de fils, des profilés de fenêtres, des composants pour la fabrication d'automobiles, des pneus, des meubles, d'une mousse pour coussins et matelas, de bâches, de joints, de films composites, de disques, de cuirs artificiels, de contenants d'emballage, de films de bande adhésive ou de revêtements.

23. Utilisation d'un matériau de moulage, tel que défini dans l'une quelconque des revendications 13 à 18, pour la fabrication de corps moulés et de films, qui rentrent directement en contact avec des hommes ou des produits alimentaires.
